# EUROPEAN PATENT SPECIFICATION

(11) **EP 3 292 869 B1**
(45) Date of publication and mention of the grant of the patent: **14.04.2021**
(21) Application number: 16789620.8
(22) Date of filing: 04.05.2016
(51) Int. Cl.: A61K 38/17, C12N 5/0789, C07K 14/705, A61K 35/28, A61P 35/00

(54) **USE OF KAL1 PROTEIN FOR CONTROLLING CELL CYCLE OF HEMATOPOIETIC STEM CELLS**
VERWENDUNG VOM KAL1-PROTEIN ZUR STEUERUNG DES ZELLZYKLUS VON HÄMATOPOIETISCHEN STAMMZELLEN UND VERWENDUNG DAVON
UTILISATION DE LA PROTÉINE KAL1 POUR CONTRÔLER LE CYCLE CELLULAIRE D'UNE CELLULE SOUCHE HÉMATOPOÏÉTIQUE

(30) Priority: 06.05.2015 KR 20150063203
(43) Date of publication of application: 14.03.2018
(73) Proprietor: Seoul National University R & DB Foundation, Seoul 08826 (KR)
(72) Inventor: KIM, Hyo-Soo, Seoul 06544 (KR); HUR, Jin, Seoul 04736 (KR); CHOI, Jae Il, Seoul 07204 (KR)
(74) Representative: Ter Meer Steinmeister & Partner
(86) International application number: PCT/KR2016/004707
(87) International publication number: WO 2016/178514

(56) References cited:
- JP-A- H11 504 803
- ANDRIES ZIJLSTRA ET AL: "The DARC side of metastasis: Shining a light on KAI1-mediated metastasis suppression in the vascular tunnel", CANCER CELL, vol. 10, no. 3, 1 September 2006 (2006-09-01), pages 177-178, XP055518552, US ISSN: 1535-6108, DOI: 10.1016/j.ccr.2006.08.012
- P KHANNA ET AL: "CD82/KAI expression prevents IL-8-mediated endothelial gap formation in late-stage melanomas", ONCOGENE, vol. 33, no. 22, 1 May 2014 (2014-05-01), pages 2898-2908, XP055518672, London ISSN: 0950-9232, DOI: 10.1038/onc.2013.249
- A. NAKAMURA-ISHIZU ET AL: "The analysis, roles and regulation of quiescence in hematopoietic stem cells", DEVELOPMENT, vol. 141, no. 24, 2 December 2014 (2014-12-02), pages 4656-4666, XP055519038, GB ISSN: 0950-1991, DOI: 10.1242/dev.106575
- BANDYOPADHYAY ET AL.: 'Interaction of KAI1 on Tumor cells with DARC on Vascular Endothelium leads to Metastasis Suppression' NATURE MEDICINE vol. 12, no. 8, 2006, pages 933 - 938, XP003012828
- TSAI ET AL.: 'Dissecting the Diverse Functions of the Metastasis Suppressor CDBVKMI' FEBS LETTERS vol. 585, no. 20, 2011, pages 3166 - 3173, XP028314401
- KOO ET AL.: 'Differential Expression of the Metastasis Suppressor KAIl in Decidual Cells and Trophoblast Giant Cells at the Feto-maternal Interface' BMB REPORTS vol. 46, no. 10, 2013, pages 507 - 512, XP055327019
- LAROCHELLE ET AL.: 'Bone Marrow Homing and Engraftment of Human Hematopoietic Stem and Progenitor Cells is mediated by a Polarized Membrane Domain' BLOOD vol. 119, no. 8, 2012, pages 1848 - 1855, XP055327036
- DATABASE NCBI [Online] 15 July 2006 XP055327247 Database accession no. AAH00726.1
- DATABASE PROTEIN [Online] 15 February 2015 XP055494713 Retrieved from NCBI Database accession no. NP_031682
- HUR ET AL.: 'CD 82/KAI1 maintains the Dormancy of Long-term Hematopoietic Stem Cells through Interaction with DARC-expressing Macrophages' CELL STEM CELL vol. 18, 17 March 2016, pages 508 - 521, XP029496783

## Description

### [Technical Field]

The present invention relates to a KAI1 protein regulating the cell cycle of hematopoietic stem cells and a use thereof, and more particularly, to a composition for regulating the cell cycle of hematopoietic stem cells including a KAI1(CD82) polypeptide or a gene encoding the polypeptide, and a pharmaceutical composition for mass culture and maintenance/storage of stem cells or preventing or treating blood tumor.

### [Background Art]

Leukemia is a type of blood tumor in which blood cells, particularly white blood cells, are abnormally grown, and therefore a large amount of improperly grown white blood cells are contained in the blood (in addition, such white blood cells including their cell organelles are much larger than normal white blood cells in size in biopsy). Compared to abnormally proliferated white blood cells, the number of normal blood cells is ultimately decreased, and therefore basic blood functions such as oxygen delivery and nutrient supply as well as immune functions cannot be performed. In addition, abnormal white blood cells have a reaction like that of an autoimmune disease, and may destroy normal tissue. Leukemia is divided into acute and chronic forms according to the degree of cell differentiation, and also divided into myelogenous leukemia and lymphocytic leukemia according to the origin of cells. Consequently, leukemia is subdivided into four large groups: i) acute myelogenous leukemia (AML); ii) chronic myelogenous leukemia (CML); iii) acute lymphocytic leukemia (ALL); and iv) chronic lymphocytic leukemia (CLL).

One method for treating leukemia is hematopoietic stem cell transplantation. Hematopoietic stem cells or hemocytoblasts are cells capable of potentially differentiating into the major components of blood. The hematopoietic stem cells can differentiate into cells produced from the bone marrow cells (monocytes, macrophages, neutrocytes, basophilic leukocytes, red blood cells, thrombocytes, etc.), lymphocytic cells (T cells, B cells, and NK cells), etc. These cells account for a relatively small proportion, making up 1/10,000 of the cells in bone marrow tissue, but have self-replicating capability and therefore can always produce the components of blood at the right time. For this reason, when an abnormality occurs in cell differentiation like leukemia, or the number of hematopoietic stem cells is reduced like aplastic anemia, these diseases may be fundamentally treated by transplantation of hematopoietic stem cells.

However, after hematopoietic stem cell transplantation, due to a long-term decrease in immune function, repeated bacterial, viral, or fungal infection may occur, and recovery of immune function may depend on the type of hematopoietic stem cell graft, the duration of immunosuppressant administration, or a graft-versus-host disease. In the case of an allograft, a graft-versus-host disease may occur and become a major factor in determining the outcome of transplantation as well as recurrence.

Therefore, minimizing such hematopoietic stem cell transplantation has become a major subject of study, but sufficient results still have not been produced.

A. Zijlstra et al., "The DARC side of metastasis: Shining a light on KAIl-mediated metastasis suppression in the vascular tunnel", CANCER CELL, US, (20060901), vol. 10, no. 3, pages 177 - 178 discloses that specific cell surface interactions between metastasis suppressors KAI1 on tumor cells and the decoy cytokine receptor DARC on adjacent vascular cells triggers senescence in the tumor cells and suppresses metastasis.

P. Khanna et al., "CD82/KAI expression prevents IL-8-mediated endothelial gap formation in late-stage melanomas", ONCOGENE, London, (20140501), vol. 33, no. 22, pages 2898 - 2908 describes that ectopic CD82 expression suppresses IL-8 secretion and that binding of CD82 tetraspanins to DARC receptors interrupts IL-8 signaling in endothelial cells.

Larochelle et al., "Bone Marrow Homing and Engraftment of Human Hematopoietic Stem and Progenitor Cells is mediated by a Polarized Membrane Domain", Blood, (20120000), vol. 119, no. 8, pages 1848 - 1855 describes a correlation between the CD82 polarity and G0 phase (quiescence) of normal human HSPCs.

### [Disclosure]

### [Technical Problem]

To solve the above-mentioned problems, the present invention is directed to the use of a composition for regulating the cell cycle of hematopoietic stem cells, the composition comprising:
a KAI1(CD82) polypeptide or a gene encoding the same, and
a DARC polypeptide or a gene encoding the same,
wherein the use is in vitro.

The present invention is also directed to a method for collecting quiescent hematopoietic stem cells, comprising:
increasing KAI1 expression in hematopoietic stem cells,
wherein the hematopoietic stem cells are long-term hematopoietic stem cells (LT-HSCs), short-term hematopoietic stem cells (ST-HSCs), or multipotent progenitors (MPPs), wherein the hematopoietic stem cells are cultured with a rhDARC, DARC positive monocyte, DARC positive macrophage, or DARC positive macrophage culture solution to increase or maintain KAI1 expression.

However, technical problems to be solved in the present invention are not limited to the above-described problems, and other problems which are not described herein will be fully understood by those of ordinary skill in the art from the following description.

### [Technical Solution]

To achieve the objects of the present invention, the present invention provides a composition which is used for regulating the cell cycle of hematopoietic stem cells in vitro, the composition comprising:
a KAI1(CD82) polypeptide or a gene encoding the same, and
a DARC polypeptide or a gene encoding the same.

In one exemplary embodiment of the present invention, the KAI1 polypeptide may consist of amino acids of SEQ ID NO: 1 or SEQ ID NO: 2.

In still another exemplary embodiment of the present invention, the DARC polypeptide may consist of amino acids of SEQ ID NO: 3 or SEQ ID NO: 4.

In yet another exemplary embodiment of the present invention, the hematopoietic stem cells may be long-term hematopoietic stem cells (LT-HSCs), short-term hematopoietic stem cells (ST-HSCs), or a multipotent progenitors (MPPs).

The present invention provides a method for harvesting quiescent hematopoietic stem cells, which includes increasing expression of KAI1 in hematopoietic stem cells, wherein the hematopoietic stem cells are LT-HSCs, ST-HSCs, or MPPs, wherein the hematopoietic stem cells are cultured with a rhDARC, DARC positive monocytes, DARC positive macrophages, or a DARC positive macrophage culture solution to increase or maintain KAI1 expression.

### [Advantageous Effects]

According to the present invention, since KAI1(CD82) can be expressed in the uppermost hematopoietic stem cells (LT-HSCs) of the hierarchy of hematopoietic stem cells and LT-HSCs can be obtained with high purity using the KAI1, various side effects of hematopoietic stem cell transplantation can be inhibited by transplanting only LT-HSCs with high purity.

Also, KAI1 significantly acts on the quiescence of LT-HSCs to sort quiescent LT-HSCs with high purity, and therefore will be a beneficial tool for studying quiescent LT-HSCs from now.

Moreover, KAI1 significantly acts on the quiescence of LT-HSCs and maintains the quiescence of LT-HSCs to have a resistance to cell damage caused by various types of stress (5-FU, irradiation, etc.). Therefore, KAI1 is expected to form a cell bank using the LT-HSCs and expected to be used as a cellular therapy product for blood tumor through cell transplantation.

### [Description of Drawings]

FIG. 1 shows the result of sorting each population of LT-HSC, ST-HSC, and MPP cells.
FIG. 2 shows the result of determining expression levels of CD9, CD37, KAI1(CD82), and CD151 using RT-PCR by isolating RNA from LT-HSCs, ST-HSCs, and MPPs.
FIG. 3 shows the result of identifying the expression of KAI1, CD9, CD37 and CD151 by staining LT-HSCs, ST-HSCs and MPPs.
FIG. 4 shows the result of additionally staining KAI1 using markers (HSC1, MPP1, MPP2), which has been reported in a recent research paper.
FIGS. 5 and 6 show the result of staining a bone of a C57BL/6 mouse to determine a location of KAI1(+)LT-HSCs in the bone.
FIGS. 7 and 8 show the result of confirming KAI1 Knock-out mouse Genomic DNA manufactured in the present invention.
FIGS. 9 to 11 show the result of comparing LT-HSC, ST-HSC, and MPP cell count in bone marrow (BM) between a wild type (WT) and KAI1 K/O mice using FACS analysis.
FIG. 12 shows the result of a colony forming unit (CFU) assay for BM cells of WT and KAI1 K/O mice using MethoCult™ GF M343.
FIG. 13 shows the result of a long-term culture-initiating cell (LTC-IC) assay to quantify LT-HSCs.
FIGS. 14 and 15 show the results of ki67 and Hoechst 33342 analyses for LT-HSCs (CD34-LSK) using LSR II after bone marrow (BM) was obtained from WT and KAI1 K/O mice.
FIGS. 16 and 17 show the results of confirming incorporation of exogenous BrdU into Genomic DNA to determine proliferative activity in WT and KAI1 K/O mice in vivo.
FIG. 18 shows the results of confirming expression of p21, p27, and p57 in LT-HSCs of WT and KAI1 K/O mice using RT-PCR.
FIG. 19 shows the result of confirming Rb phosphorylation in LT-HSCs of WT and KAI1 K/O mice using FACS analysis.
FIG. 20 shows the result of comparing gene expression of KAI1 in Lin(-)CD34(-) cells and Lin(-)CD34(+) cells.
FIG. 21 shows the result of confirming KAI1 knock-down by qRT-PCR after KAI1 knock-down is induced in EML cells using a shRNA construct.
FIG. 22 shows the result of confirming the increase in KAI1 expression by qRT-PCR after KAI1 is over-expressed in EML cells.
FIGS. 23 and 24 shows the results of RNA sequencing analysis for KAI1 knock-down and KAI1 overexpression EML cells.
FIGS. 25 and 26 show the results of confirming the increase/decrease in TGF-β1 and TGFR2 expression in KAI1 knock-down and KAI1 overexpression EML cells using RT-PCR and Western blotting.
FIGS. 27 and 28 show the result of comparing TGF-β1 and TGFR2-positive cells in LT-HSCs of WT and KAI1 K/O mice.
FIGS. 29 and 30 show the results of determining TGF-β1 expression and secretion using Western blotting after KAI1 overexpression EML cells are treated with an inhibitor.
FIG. 31 shows the result of comparing phosphorylation of Smad2 and Smand3 as downstream pathways of TGF-β1/TGFR and the increase/decrease in expression of CDK inhibitors such as p21, p27, and p57 in KAI1 knock-down and KAI1 overexpression EML cells using Western blotting.

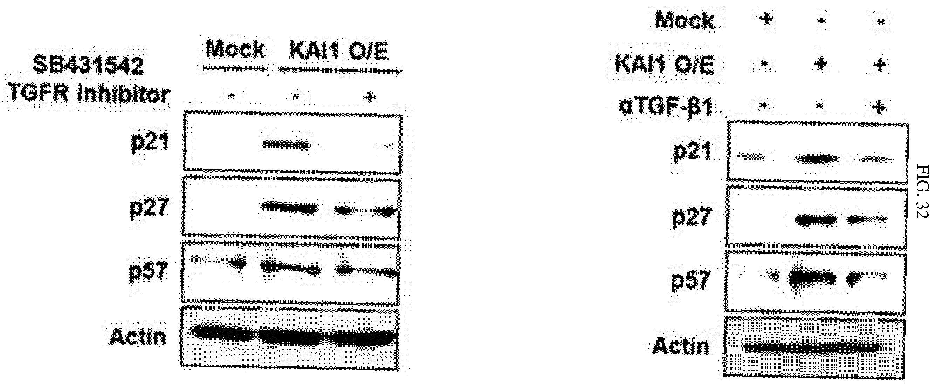

FIG. 32 shows the result of determining the increase/decrease in p21, p27, and p57 expression using Western blotting after KAI1 overexpression EML cells are treated with a TGFR inhibitor or a neutral antibody against TGF-β1.
FIGS. 33 and 34 show the results of comparing the cell cycles of Mock/, KAI1 O/E EML using FACS.
FIGS. 35 to 38 show continuous changes in KAI1-positive LT HSCs and proliferating blood cells in BM by injecting 5-FU into WT mice, following sub-lethal irradiation of the WT mice.
FIGS. 39 and 40 show the results of recovery of total BM or LSK cells in BM cells of KAI1^{-/-} and WT mice following sub-lethal irradiation.
FIG. 41 shows a schematic diagram illustrating a competitive BM transplantation (BMT) experiment to examine whether KAI1 affects long term repopulating capacity of HSCs.
FIG. 42 shows the result of FACS analysis of BM cells obtained according to the process illustrated in FIG. 41.
FIGS. 43 and 44 show the results of comparing LSK and LT-HSCs in BM cells of mice receiving HSPCs of KAI1^{-/-} mice and BM cells of mice receiving HSPCs of WT mice at the second BMT in serial BMT.
FIGS. 45 and 46 show the results of comparing cell cycles of CD34- LSK cells in mice receiving HSPCs derived from KAI1^{-/-} mice and mice receiving HSPCs derived from WT.
FIG. 47 shows the result of the Western blotting of a duffy antigen receptor for chemokines (DARC) following immunoprecipitation in EML cells using a KAI1 antibody.
FIG. 48 shows the result of identifying F4/80(+) cells or Lin(-) cells using a magnetic-activated cell sorting (MACS) technique after being co-cultured and then stained with KAI1 and DARC.
FIGS. 49A and 49B show the results of fluorescence staining after paraffin blocks of a femur of a C57 mouse (FIG. 49A) or a femur of a Tie2-GFP mouse (FIG. 49B) are prepared, sectioned, and then subjected to deparaffinization and a retrieval process to confirm in vivo interaction between KAI1 and DARC.
FIG. 50 shows the result of confirming DARC expression in endothelial cells, stromal cells, and monocytes/macrophages to identify the type of DARC(+) cells.
FIG. 51 shows the result of comparing degrees of KAI1 expression in Lin(-)CD34(-) and Lin(-)CD34(+).
FIG. 52 shows the result of confirming PKC phosphorylation caused by KAI1 activity.
FIG. 53 shows the result of confirming that rhDARC improves PKCa phosphorylation by KAI1.
FIG. 54 shows the result of analyzing cell cycles after qEML cells are co-cultured with Mock Raw 264.7 cells, DARC Knock-down Raw 264.7 cells, and DARC Knock-down Raw 264.7 cells, pretreated with rhDARC, and cultured for 2 days.
FIG. 55 shows the result of confirming KAI1(+) cell population after being sorted by MACS using Lin(-)CD34(-) cells and then cultured with a stem cell factor (SCF).
FIGS. 56 and 57 show the results of confirming DARC expression in Raw 264.7 cells in stress or mobilization niche such as G-CSF or 5-FU.
FIG. 58 shows the result of confirming, following reduction of DARC expression in Raw 264.7 cells using shRNA, KAI1 expression when Mock Raw264.7 and DARC Knock-down Raw264.7 cells are co-cultured and SCF is added to quiescent EML cells sorted from the total EML cells.
FIG. 59 shows the result of confirming KAI1 expression when Mock and DARC Knock-down Raw 264.7 supernatants were co-cultured with EML cells to examine a paracrine effect.
FIG. 60 shows the result of confirming KAI1 expression after qEML cells are sorted from EML cells and cultured with SCF.
FIG. 61 shows the result of Western blotting for mono/poly-ubiquitin after EML cells are cultured with SCF, and 8 hours later, subjected to KAI1 immunoprecipitation (IP).
FIGS. 62 to 64 show the results of confirming KAIl-biotin conjugation after all groups of qEML only, qEML + Mock Raw 264.7 cells, qEML + DARC Knock-down Raw 264.7 cells, qEML + DARC Knock-down Raw 264.7 + rhDARC cells, and MG-132-pretreated qEML cells were cultured with SCF for 3 hours, membrane-binding antibodies are eliminated with acidic buffer (50 mM Glycine, 100 mM NaCl, pH 2.5), and endocytosis is induced.
FIGS. 65 and 66 show the result of FACS analysis for human LT-HSC after being stained with LIN, CD38, CD34, CD93, and CD45RA antibodies.
FIG. 67 shows the result of fluorescence staining after MNCs are obtained from human umbilical cord blood, and then Lin(+) CD235(+)CD235(+) cells are eliminated by MACS (first step), and KAI1(+)LT-HSC and KAI1(-)LT-HSC are sorted by FACS, subjected to starvation for 3 hours, treated with rhDARC, and subjected to fixation/permeabilization.
FIG. 68 shows the result of analyzing cell cycles after MNCs are obtained, Lin(+)CD235(+)DARC(+) cells are eliminated, subjected to starvation for 3 hours, and then treated with rhDARC for 2 days to examine whether quiescence of KAI1(+)LT-HSC is increased due to rhDARC.
FIG. 69 shows the result of confirming DARC expression in CD14(+) cells of human umbilical cord blood.
FIG. 70 shows the result of obtaining Lin(-)CD235(-)DARC(-) cells from umbilical cord blood, subjecting some of the same umbilical cord blood to MACS to sort un-touched monocytes, and then culturing [SCF, Flt3-ligand, TPO, IL-3] HSCs only by using a growth factor.
FIG. 71 shows the summary of the results of the examples.

### [Modes of the Invention]

As a result of research on a method for reducing side effects of hematopoietic stem cell transplantation, the inventors first found a marker which is called KAI1(CD82) expressed only in the uppermost LT-HSCs of the hierarchy of hematopoietic stem cells. In addition, it was confirmed that KAI1 significantly acts on the quiescence of LT-HSCs and maintains the quiescence of LT-HSCs, thereby having a resistance to various types of stress (5-FU, irradiation, etc.). Based on this, the present invention was achieved.

Hereinafter, the present invention will be described in detail.

The present invention provides a composition for use in regulating the cell cycle of hematopoietic stem cells in vitro, which includes a KAI1(CD82) polypeptide or a gene encoding the same, wherein the hematopoietic stem cells are preferably LT-HSCs, ST-HSCs or MPPs. In addition, the composition for use of the present invention further includes a DARC polypeptide or a gene encoding the same.

The present invention may regulate the cell cycle of the LT-HSCs by regulating the expression of the KAI1(CD82) protein which is only specifically expressed in the LT-HSCs. That is, as the expression of the KAI1(CD82) protein is increased, the G0 phase (quiescence) of the LT-HSCs may be maintained due to the increase in TGF-β1 secretion via PKC, and as expression of the KAI1(CD82) protein is decreased, proliferation and differentiation of the LT-HSCs may be promoted via a reverse signaling mechanism.

In the present invention, the KAI1(CD82) polypeptide may consist of a human-derived amino acid sequence of SEQ ID NO: 1 or a mouse-derived amino acid sequence of SEQ ID NO: 2, but the present invention is not limited thereto. The KAI1(CD82) polypeptide may include a protein represented by an amino acid sequence having 70% or more, preferably 80% or more, more preferably 90% or more, and most preferably 95% or more homology with the amino acid sequence. In addition, in the present invention, the gene may be any gene capable of encoding a KAI1 polypeptide having an amino acid sequence of SEQ ID NO: 1 or SEQ ID NO: 2.

In one exemplary embodiment of the present invention, it was confirmed that KAI1(CD82) is specifically expressed only in LT-HSCs of LT-HSCs, ST-HSCs and MPPs (refer to Example 1). In addition, as a result of confirming that the cell cycle of the LT-HSCs is regulated according to KAI1(CD82) expression, compared to WT, in LT-HSCs of a KAI1 K/O mouse, G0 phase was reduced whereas G1/S/G2 phases were extended, and the number of LT-HSCs was also decreased (refer to Examples 2 and 3). In addition, it was confirmed that KAI1 increases expression and secretion of TGF-β1 via PKC, not Erk (refer to Example 4).

From the above-mentioned experimental results, it can be seen that the cell cycle and differentiation of hematopoietic stem cells, particularly LT-HSCs, may be regulated by regulating KAI1 expression, which is effective against blood tumor. Therefore, a material for improving KAI1 or KAI1 expression may maintain the quiescence of hematopoietic stem cells may also be effective in treating blood tumor.

Therefore, in another aspect of the present invention, the present disclosure provides a pharmaceutical composition for preventing or treating blood tumor, which includes a KAI1(CD82) polypeptide or a gene encoding the same, and may further include a DARC polypeptide or a gene encoding the same.

The term "prevention" used herein refers to all actions of inhibiting the development of blood tumor or delaying the onset of the blood tumor by administration of the pharmaceutical composition according to the present disclsoure.

The term "treatment" used herein refers to all actions involved in alleviating or beneficially changing symptoms of blood tumor by administration of the pharmaceutical composition according to the present disclosure.

The term "blood tumor," which is a disease to be ameliorated, prevented, or treated by the composition of the present disclosure, may be a malignant tumor occurring in white blood cells or a lymph system, and selected from the group consisting of lymphoma, multiple myeloma, myelogenous leukemia, and lymphocytic leukemia, but the present disclosure is not limited thereto.

The pharmaceutical composition for use of the present invention may further include a pharmaceutically acceptable carrier. The pharmaceutically acceptable carrier may include saline, polyethyleneglycol, ethanol, vegetable oil, isopropylmyristate, etc., but the present invention is not limited thereto.

The composition for use of the present invention, which includes a KAI1 polypeptide or gene encoding the same as an active ingredient, may include the active ingredient at 0.0001 to 50 wt% with respect to the total weight.

A preferable dosage of the pharmaceutical composition for use of the present invention may be determined by one of ordinary skill in the art according to a condition and body weight of an individual, severity of a disease, a drug form, an administration route, and duration. However, the pharmaceutical composition of the present invention is preferably administered at 0.001 to 100 mg/kg, and more preferably 0.01 to 30 mg/kg a day. The pharmaceutical composition for use the present invention may be administered once or several times a day.

The pharmaceutical composition for use of the present invention may be administered into a mammal such as a rat, a mouse, a stock, or a human via various routes. All methods of administration may be expected, and the pharmaceutical composition for use of the present invention may be administered, for example, orally, or by rectal, intravenous, intramuscular, subcutaneous, epidural, or intracerebroventricular injection.

The pharmaceutical composition for use of the present invention may be prepared in various pharmaceutical forms, and thus there is no limit to a drug form.

In still another aspect, the present disclosure provides a method for preventing or treating blood tumor by administering a pharmaceutically effective amount of the pharmaceutical composition into a subject. The term "subject" refers to a target to be treated, and more specifically, a mammal such as a human, a non-human primate, a mouse, a rat, a dog, a cat, a horse, and a cow. In addition, it is apparent to those of ordinary skill in the art that the "pharmaceutically effective amount" used herein may be controlled in various ranges according to a body weight, age, sex, or health condition of a patient, a diet, an administration time, an administration route, an excretion rate, and a severity of a disease.

In addition, according to the present invention, the quiescence of LT-HSCs may be maintained by the expression of KAI1(CD82), and thereby the LT-HSCs may have a resistance to various types of stress.

In another exemplary embodiment of the present invention, it was confirmed that a KAI1(CD82)-expressed mouse group has a resistance to 5-FU and irradiation (refer to Example 5).

Therefore, in yet another aspect, the present disclosure provides a method for purifying hematopoietic stem cells having a resistance to stress, which includes the following steps:
(a) analyzing KAI1 expression in an isolated hematopoietic stem cell population; and
(b) isolating and collecting KAI1-expressed hematopoietic stem cells.

Meanwhile, in yet another exemplary embodiment of the present disclosure, as a result of examining a mechanism for regulating a ligand associated with KAI1 activity and KAI1 expression, it was confirmed that KAI1 interacts with DARC and expression is regulated by co-culture with rhDARC, DARC positive monocytes, or DARC positive macrophages (refer to Examples 7 and 8).

Therefore, in yet another aspect of the present invention, the present invention provides a method for collecting quiescent hematopoietic stem cells, which includes increasing KAI1 expression in hematopoietic stem cells, wherein the hematopoietic stem cells are LT-HSCs, ST-HSCs, or MPPs. Here, the KAI1 expression may be increased or maintained by culturing the hematopoietic stem cells with a rhDARC, DARC positive monocyte, DARC positive macrophage, or DARC positive macrophage culture solution.

In the present invention, the DARC polypeptide may consist of a human-derived amino acid sequence of SEQ ID NO: 3 or a mouse-derived amino acid sequence of SEQ ID NO: 4, but the present invention is not limited thereto. The DARC polypeptide may include a protein represented by an amino acid sequence having 70% or more, preferably 80% or more, more preferably 90% or more, and most preferably 90% or more homology with the amino acid sequence.

In yet another aspect, the present disclosure provides quiescent hematopoietic stem cells collected by the above-described method. The quiescent hematopoietic stem cells of the present invention may increase in vivo transplantation efficiency, proliferation ability, differentiation ability, or stability, and may improve pancreatic transplantation, organ transplantation such as liver regeneration, or regeneration ability.

Hereinafter, to help in understanding the present invention, exemplary embodiments will be disclosed. However, the following examples are merely provided to more easily understand the present invention, and the scope of the present invention is not limited to the examples.

### [Examples]

### Example 1. Identification of KAI1(CD82) as maker of LT-HSCs

To examine whether KAI1(CD82) becomes a marker of the uppermost hematopoietic stem cells (LT-HSCs) in the hierarchy of hematopoietic stem cells, an experiment was carried out as follows:

### 1-1. Collection of cells

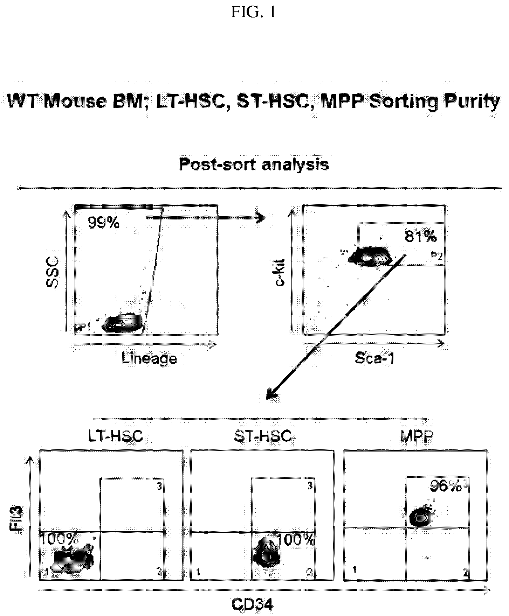

First, to obtain LT-HSCs, ST-HSCs, or MPPs, BM cells were obtained from femurs and tibias of C57BL/6 mice, and then peripheral blood mononuclear cells (PBMCs) were isolated therefrom using Histopaque-1083 (Sigma-Aldrich). In addition, the isolated PBMCs were stained using lineage cells (CD3e, CD11b, CD45R/B220, Erythroid Cells, Ly-6G, and Ly-6C), and c-kit, Sca-1, Flt3, CD34 antibodies. With well-known conventional markers, the LT-HSCs were designated as Flt3-CD34-LSK, the ST-HSCs were designated as Flt3-CD34+LSK, and MPPs were designated as Flt3+CD34+LSK, wherein the LSK indicates Lineage(-)Sca-1(+)c-kit(+). Each population was determined using an FACS Arial flow cytometer, and consequently, as shown in FIG. 1, it can be confirmed that each population was sorted with high purity.

### 1-2. Confirmation of KAI1(CD82) expression in LT-HSCs

To confirm whether KAI1(CD82) was only expressed in the LT-HSCs, first, RNA of the cells obtained in Example 1-1 was isolated to determine expression levels of CD9, CD37, KAI1(CD82), and CD151, which are known to be tetraspanin, using RT-PCR. Primer information used herein is shown in Table 1.

**[Table1]**

| Type | Primer sequence | |
|---|---|---|
| CD9 | FW (Forward) | 5'-AGTGCATCAAATACCTGCTCTTC-3 ' (SEQ ID NO: 5) |
| | RV (Reverse) | 5'-CTTTAATCACCTCATCCTTGTGG-3 ' (SEQ ID NO: 6) |
| CD37 | FW (Forward) | 5'-CTTCGTTTTCAACCTCTTCTTCT-3 ' (SEQ ID NO: 7) |
| | RV (Reverse) | 5'-AACTGTGCATAGTCCCAACTCTC-3 ' (SEQ ID NO: 8) |
| CD81 | FW (Forward) | 5'-TTCTACGTGGGCATCTACATTCT-3 ' (SEQ ID NO: 9) |
| | RV (Reverse) | 5'-GCTGTTCCTCAGTATGGTGGTAG-3 ' (SEQ ID NO: 10) |
| CD151 | FW (Forward) | 5'-TGCCTCAAGTACCTGCTCTTTAC-3 ' (SEQ ID NO: 11) |
| | RV (Reverse) | 5'-CTGACTGGTGGTATCTCTTGACC-3 ' (SEQ ID NO: 12) |
| KAI1 (CD82) | FW (Forward) | |
| | RV (Reverse) | |

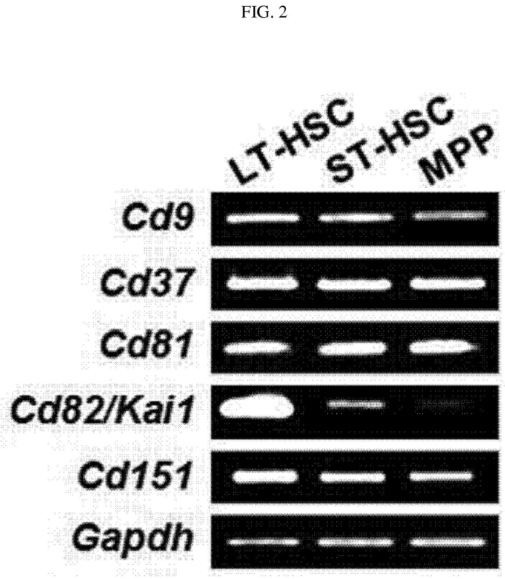

As shown in FIG. 2, it can be seen from the result that most of the tetraspanins were expressed in all the LT-HSCs, ST-HSCs and MPPs, but the KAI1(CD82) was only specifically expressed in the LT-HSCs.

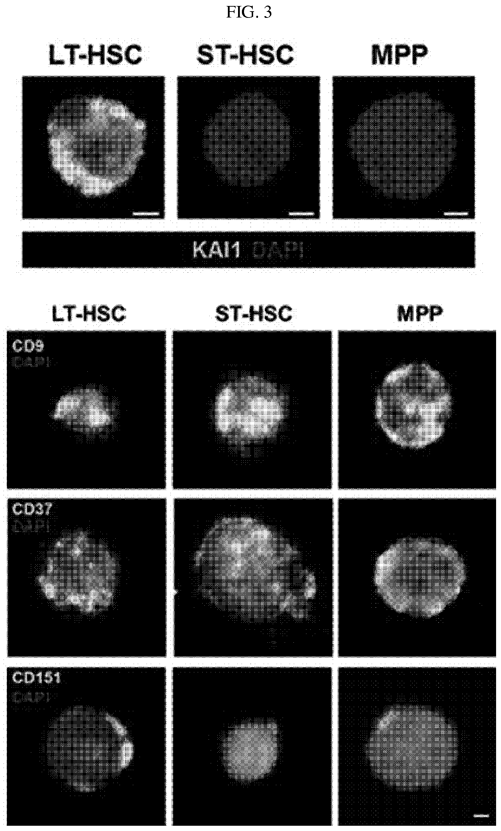

Subsequently, fluorescence staining was performed on the cells obtained in Example 1-1, and therethrough, as shown in FIG. 3, it can be seen that the KAI1 was only expressed in the LT-HSCs while CD9, CD37, and CD151 were expressed in all of the LT-HSCs, ST-HSCs, and MPPs.

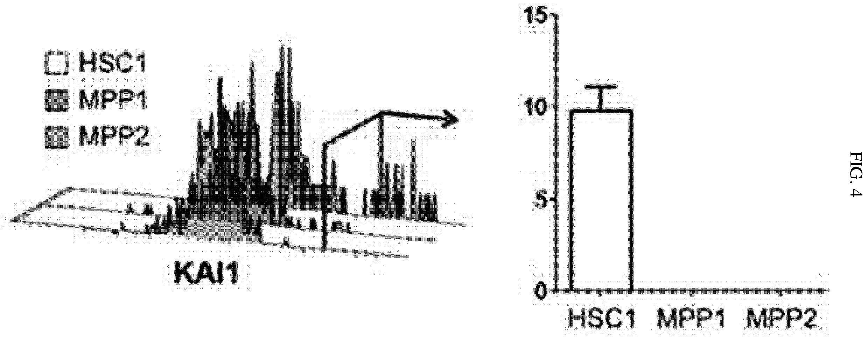

Moreover, in a recently published research paper, using quiescent HSC markers with a higher purity, HSC1 or quiescent HSC was designated as CD48-CD229-CD244-CD150+LSK, MPP1 was designated as CD48-CD229-CD244-CD150-LSK, and MPP2 was designated as CD48-CD229+CD244-CD150-LSK, and as shown in FIG. 4, staining of KAI1 with the same markers used previously shows that approximately 10% of KAI1(+) cells were expressed in HSC1 (quiescent HSC) but no KAI1(+) cells were expressed in MPP1 and MPP2.

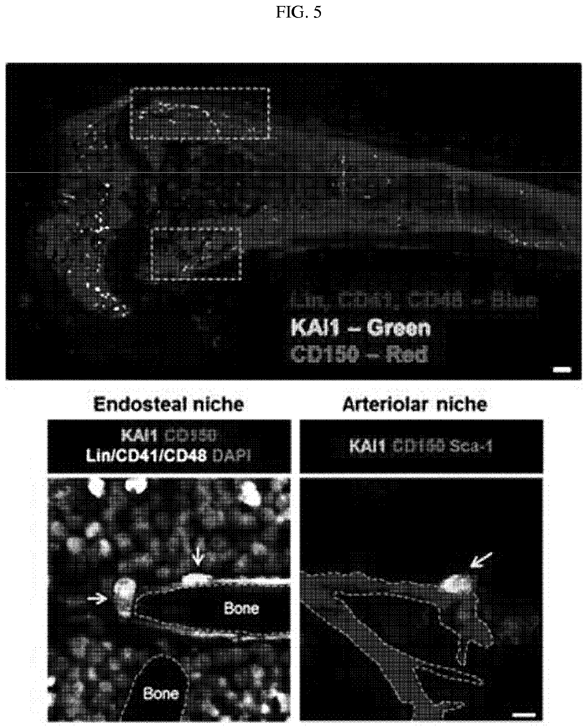

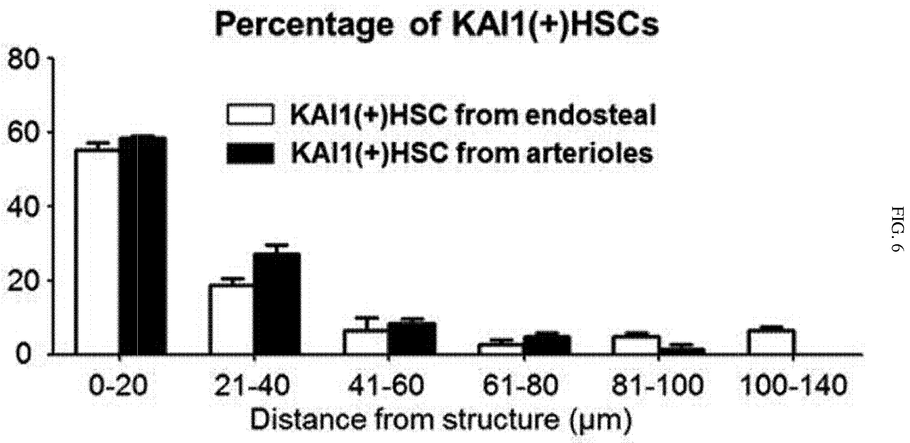

Furthermore, to determine the location of KAI1(+)LT-HSC in a bone, the bone of a C57BL/6 mouse was stained. Bone staining was performed by fixing the femur of the C57BL/6 mouse with 4% paraformaldehyde for 24 hours. Afterwards, the resulting femur of the C57BL/6 mouse was exposed to a decalcification solution at room temperature for 24 hours to prepare a slide using a paraffin block. The paraffin slide was stained through a deparaffinization process. As a result of examination of cells in which lineage, CD41 and CD48 as LT-HSC markers, and CD150 and KAI1 as negative markers were simultaneously expressed, as shown in FIGS. 5 and 6, it can be seen that most of the cells were located in bone linings. More specifically, most of Lin(-)CD41(-)CD48(-)CD150(+)KAI1(+) cells were located in the endosteal niche and the arteriolar niche. Meanwhile, according to the prior references, the endosteal niche and the arteriolar niche are known as places retaining quiescent HSCs.

### Example 2. Confirmation of increase in LT-HSC cell count in bone-marrow (BM) according to KAI1(CD82) expression

### 2-1. Preparation of KAI1 knock-out mice

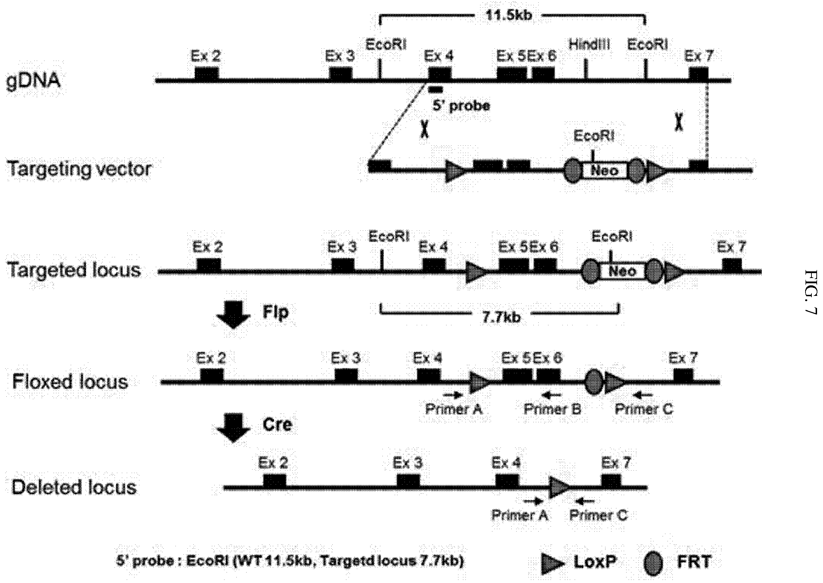

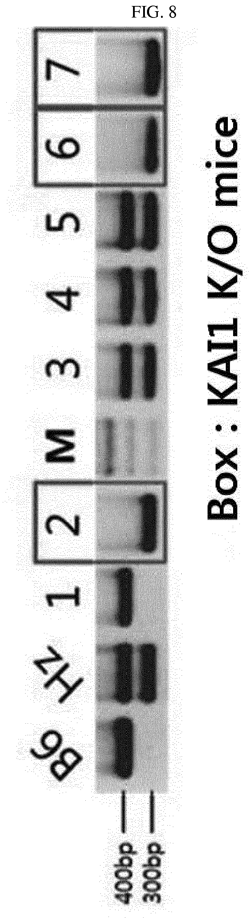

The inventors themselves were the first in the world to prepare KAI1 knock-out mice . More specifically, the KAI1 knock-out mouse was prepared by targeting the 5^{th} and 6^{th} genes of KAI1, and then identified using mouse genomic DNA and primers listed in Table 2 below (refer to FIGS. 7 and 8). In the drawings, 400bp represents WT, and 300bp represents the KAI1 K/O mouse.

**[Table 2]**

| Type | Primer sequence |
|---|---|
| Primer A | 5'-GGGTCCCCTAGGAAATTCAA-3' (SEQ ID NO: 15) |
| Primer B | 5'-ATGATGCAGATGTTCTCTCAGGGTG-3' (SEQ ID NO: 16) |
| Primer C | 5'-ACAGGGGACTCACCC TACAAGG-3' (SEQ ID NO: 17) |

### 2-2. Comparison of cell counts in BM between WT and KAI1 K/O mice

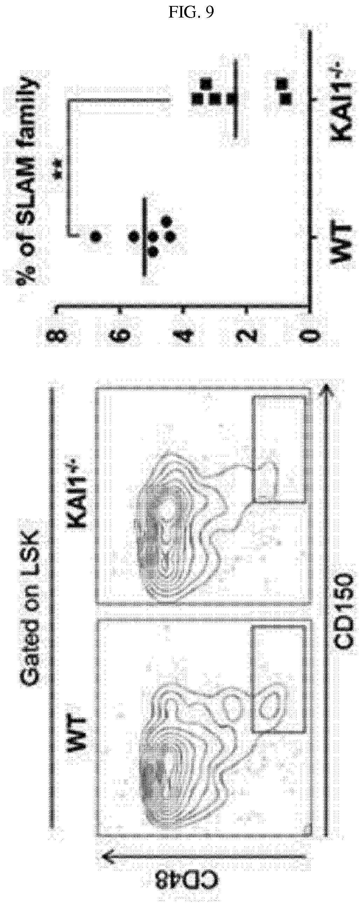

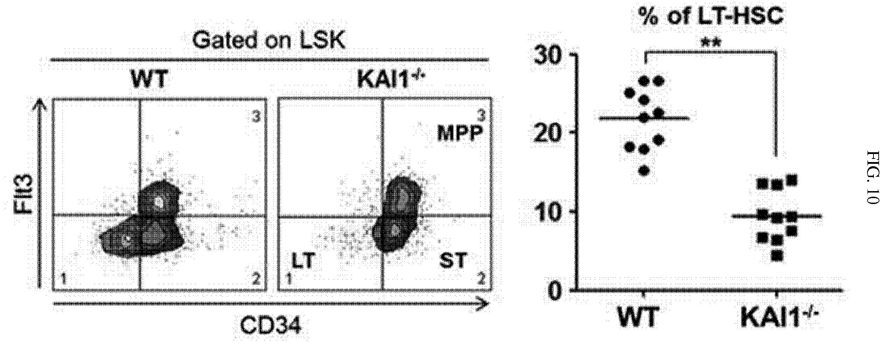

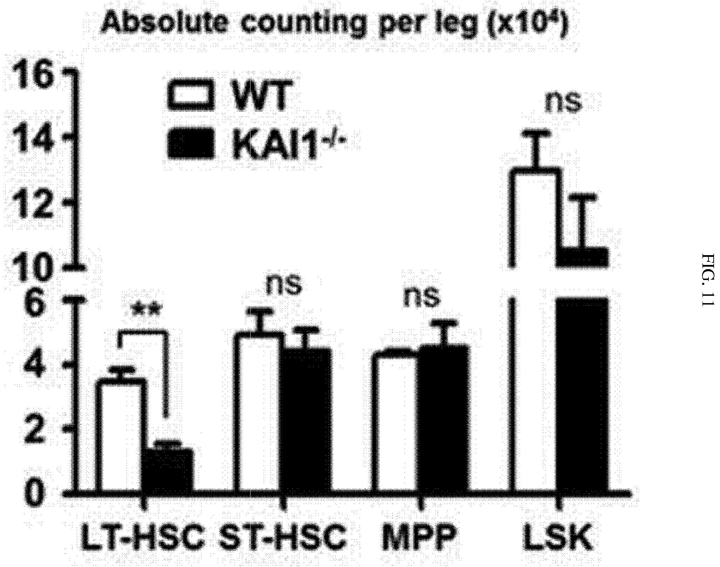

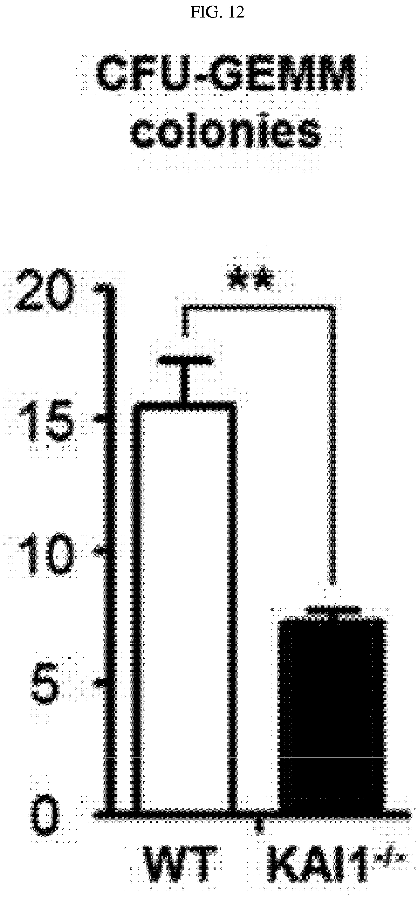

First, LT-HSC, ST-HSC, and MPP cell counts in the BM between the WT and the KAI1 K/O mice were compared using FACS analysis, showing that, as shown in FIG. 9, a cell count of CD48(-)CD150(+)LSK, which is a LT-HSC marker, was lower in the KAI1 K/O mice than in the WT. In addition, as shown in FIG. 10, a cell count of CD34(-)Flt(-)LSK, also known as an LT-HSC marker, was lower in the KAI1 K/O mice than the WT, and as shown in FIG. 11, the absolute cell counts were lower in the KAI1 K/O mice. Subsequently, a colony forming unit (CFU) assay was carried out on BM cells of WT and KAI1 K/O mice using MethoCult™ GF M343 (Stem Cell Technologies, Cat No. 03434), and therethrough, as shown in FIG. 12, it can be confirmed that multi-potential granulocytes, erythroids, macrophages, and megakaryocyte progenitors (CFU-GEMM) were reduced in the KAI1 K/O mice than WT.

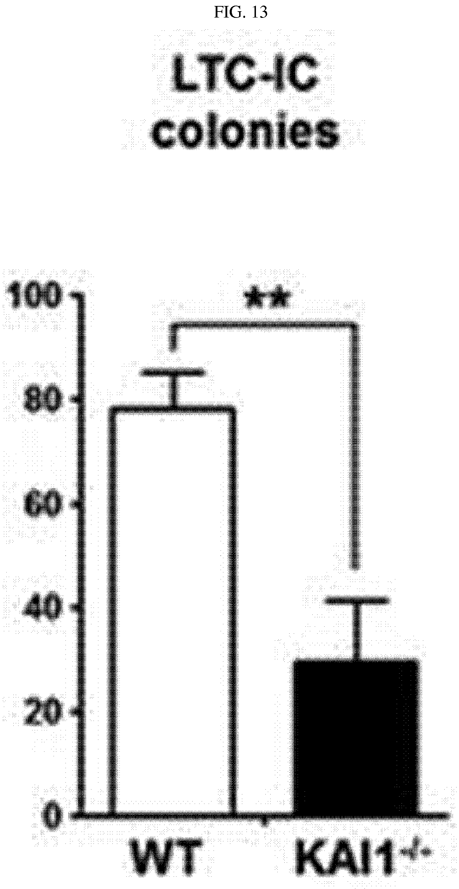

Afterwards, to quantify LT-HSCs, a long term culture-initiating cell (LTC-IC) assay was carried out. More specifically, mouse stromal cells (OP9) were exposed to irradiation at 30 Gy and used as feeder cells. In addition, LSK cells were sorted from BM in each of WT and the KAI1 K/O mice, and each cell was cultured on the feeder cell. The culturing was carried out in a hydrocortisone-containing MyeloCult medium (StemCell technology), and half-media changes were carried out once a week. After a total of four weeks of culturing, a CFU assay was carried out, and 10 days later, colony counting was performed. Therethrough, as shown in FIG. 13, it can be confirmed that a colony count was lower in the KAI1 K/O mice than in WT. Consequently, it can be seen that the LT-HSC cell count was decreased in the KAI1 K/O mice.

### Example 3. Confirmation of cell cycle of LT-HSCs according to KAI1(CD82) expression

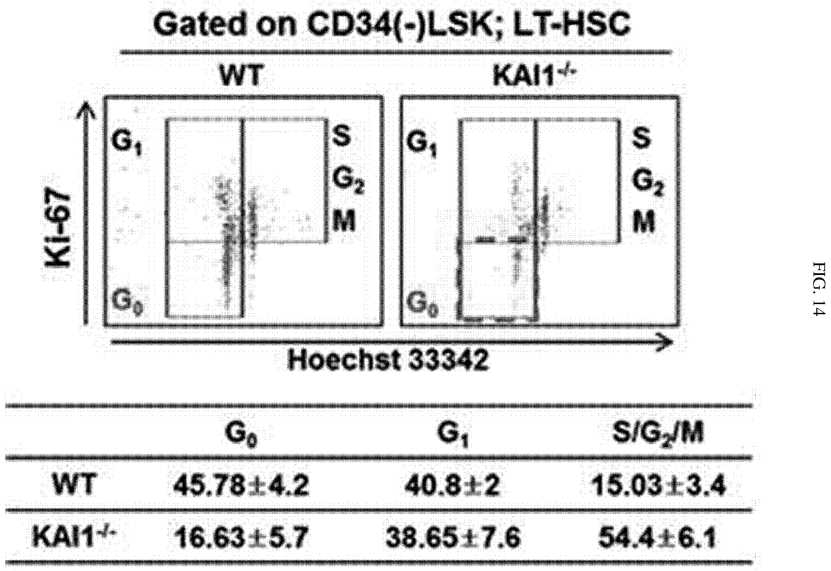

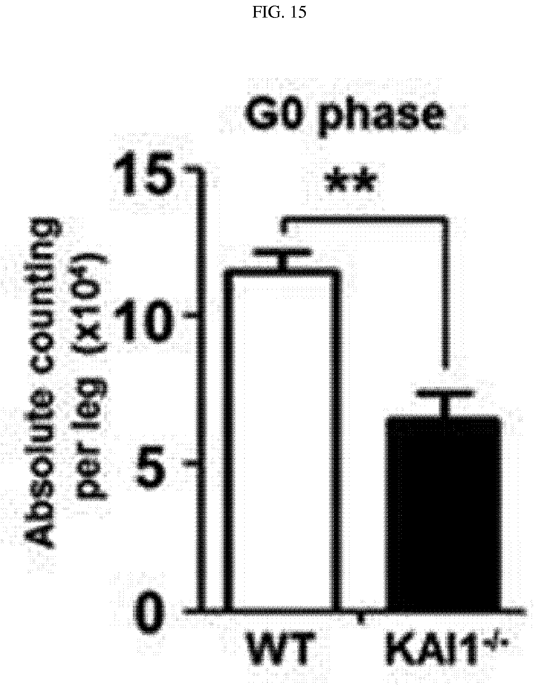

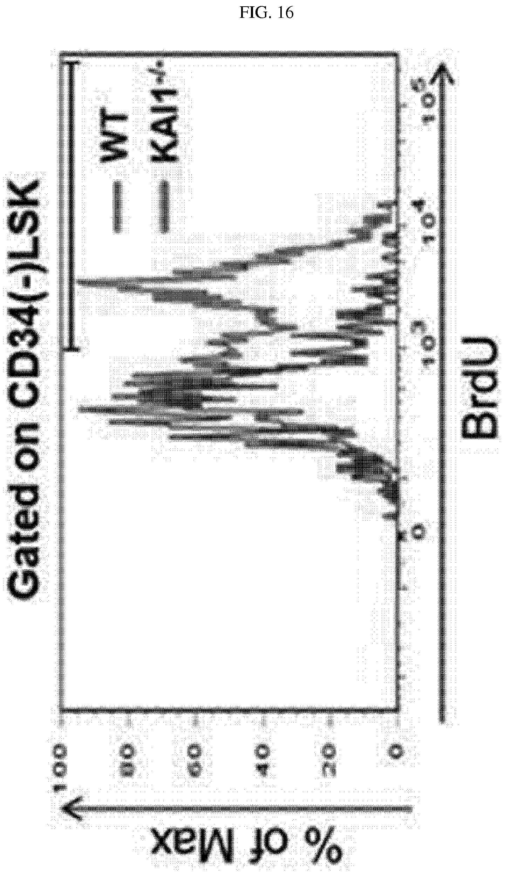

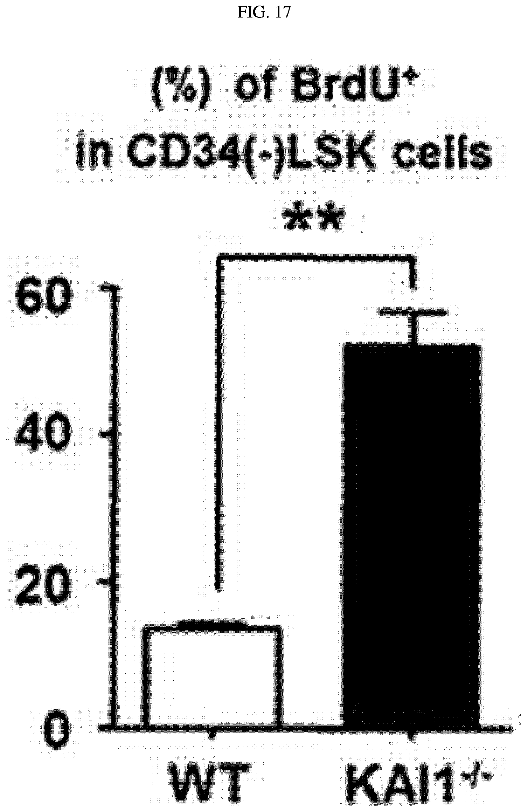

First, bone marrow (BM) of WT and KAI1 K/O mice were obtained and subjected to ki67 and Hoechst 33342 analyses of LT-HSCs (CD34-LSK) using LSR II. Consequently, as shown in FIGS. 14 and 15, it was confirmed that LT-HSCs in G0 phase were less numerous but LT-HSCs in G1/S/G2 phases were more numerous in the KAI1 K/O mice than in WT. Subsequently, to determine in vivo proliferative activity in WT and KAI1 K/O mice, exogenous BrdU incorporation into genomic DNA was examined. That is, following BrdU treatment, BM cells were obtained from each mouse to detect BrdU positive LT-HSCs, which were considerably more numerous in the KAI1 K/O mice than in WT, as shown in FIGS. 16 and 17.

Afterwards, to detect CDK inhibitors (p21, p27 and p57), which are significant genes for maintaining of HSCs, LT-HSCs of WT and the KAI1 K/O mice were sorted and then had RNA isolated therefrom for RT-PCR. Primer information used herein is listed in Table 3 below.

**[Table 3]**

| Type | Primer sequence | |
|---|---|---|
| P21 | FW (Forward) | 5'-GAGAACGGTGGAACTTTGACTTC-3 ' (SEQ ID NO: 18) |
| | RV (Reverse) | 5' -GTGA T AGAAA TCTGTCAGGCTGGT -3 (SEQ ID NO: 19) |
| P27 | FW (Forward) | 5' -CA TGAAGAACT AACCCGGGACT -3 (SEQ ID NO: 20) |
| | RV (Reverse) | 5' -CAGAGTTTGCCTGAGACCCAA T -3 ' (SEQ ID NO: 21) |
| P57 | FW (Forward) | 5'-AGATCTGACCTCAGACCCAATTC-3 ' (SEQ ID NO: 22) |
| | RV (Reverse) | 5'-GCTCTTGATTCTCGTCCTGCTC-3 ' (SEQ ID NO: 23) |

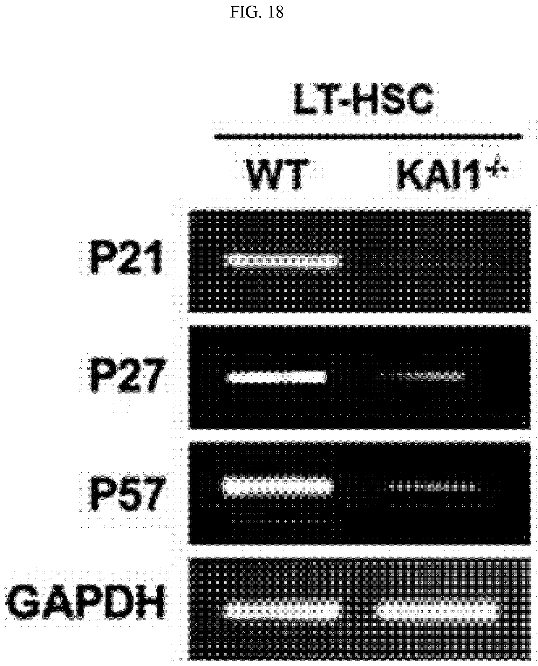

As a result, it can be seen that CDK inhibitors p21, p27, and p57 of LT-HSCs of KAI1 K/O mice were reduced compared to those of WT, as shown in FIG. 18.

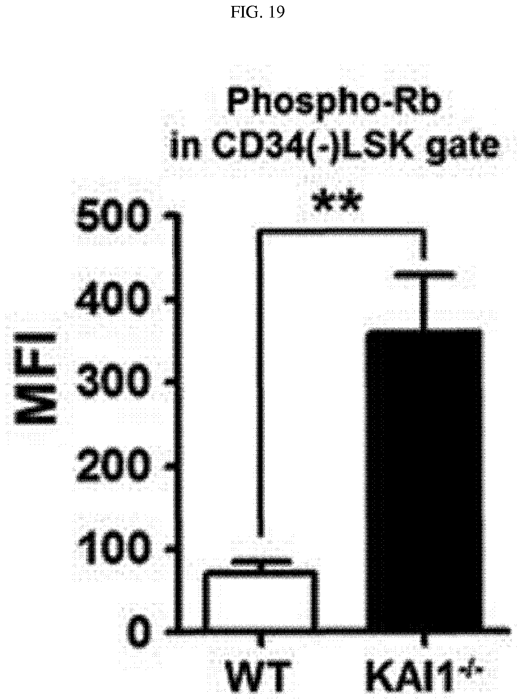

Generally, CDK inhibitors are known to stop the cell cycle by reducing Retinoblastoma (Rb) phosphorylation. Therefore, as a result of confirmation of the Rb phosphorylation of LT-HSCs in WT and the KAI1 K/O mice using FACS analysis, it can be confirmed that Rb phosphorylation was higher in the KAI1 K/O mice than in WT, as shown in FIG. 19.

From the above results, it can be seen that quiescence was reduced in the KAI1 K/O mouse LT-HSCs.

### Example 4. Identification of signaling mechanism for KAI1(CD82) to maintain quiescence of LT-HSCs

### 4-1. Preparation of cell line

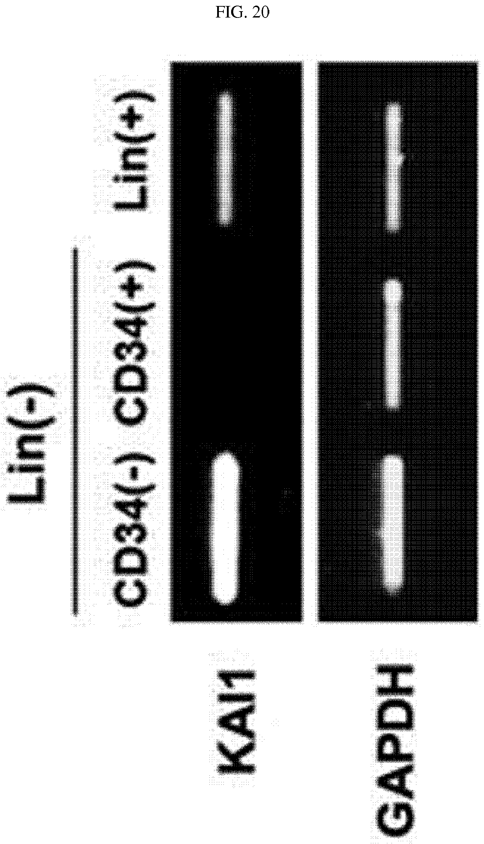

To study a mechanism of KAI1 for maintaining quiescence of LT-HSCs, an EML cell line which has been widely used in research on hematopoietic stem and progenitor cells (HSPCs) was used. The EML cell line was quiescent Lin(-)CD34(-) cells, and Lin(-)CD34(+) cells are cells exhibiting high proliferative activity, in which the cell cycle stays active. The two populations were sorted to compare KAI1 gene expression using the KAI1 primers listed in Table 1, and as a result, it can be confirmed that KAI1 was highly expressed in quiescent Lin(-)CD34(-) cells but not expressed in Lin(-)CD34(+) cells, as shown in FIG. 20.

### 4-2. KAI1 knock-down or overexpression in EML cells

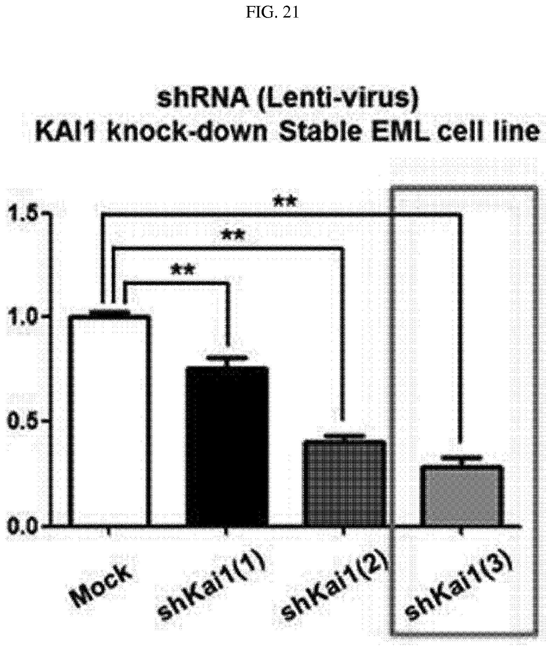

Knock-down or overexpression of KAI1 was carried out in EML cells using a lentivirus. First, shRNA constructs were used for KAI1 knock-down (Sigma-Aldrich, TRCN0000042409, TRCN0000042410, and TRCN0000042411). After transduction, selection was carried out with puromycin (2 µg/ml). In addition, the KAI1 knock-down was confirmed using real-time PCR (qPCR), and information on KAI1 primers only for qPCR used herein is listed in Table 4 below. As a result, it can be confirmed that KAI1 was knocked down in EML cells by treatment with shRNA constructs, as shown in FIG. 21.

**[Table 4]**

| Type | Primer sequence | |
|---|---|---|
| KAI1 | FW (Forward) | 5'-GCCTGGGACTACGTGCAG-3' (SEQ ID NO: 24) |
| | RV (Reverse) | 5'-CCTCGTTCTCTGTCCAGTTGT-3' (SEQ ID NO: 25) |

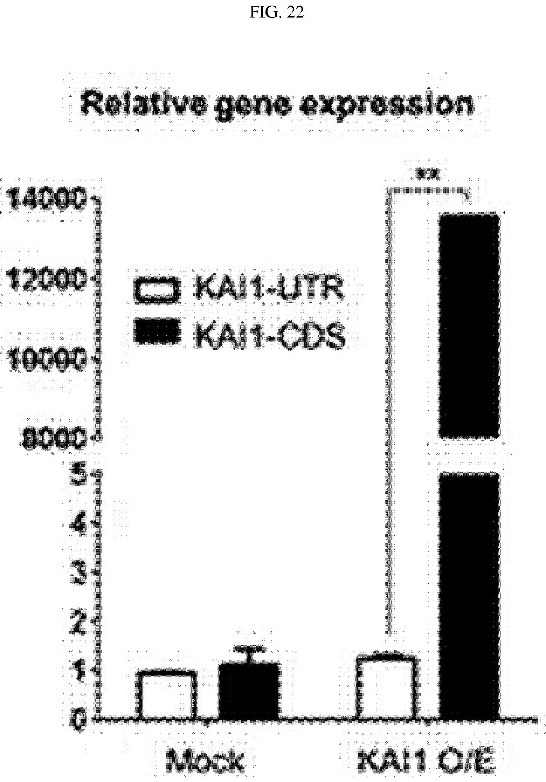

Afterwards, for KAI1 overexpression (O/E), KAI1 cDNA was inserted into pLenti 6.3/v5-Dest (Invitrogen) and then cloning was performed. 293FT cells were transfected with the KAI1 pLenti 6.3/V5-Dest using a ViraPower packaging mix (Invitrogen) and polyethylenimine (PEI). In addition, after 48 hours, viral supernatants were acquired and passed through a filter to concentrate viruses using ultracentrifugation. The viruses obtained thereby were transduced to the EML cells, and after 48 hours, selection was carried out using blasticidin S (5 µg/ml). As a result of examining KAI1 increase using the primers listed in Table 4, it can be confirmed that KAI1 was overexpressed, as shown in FIG. 22.

### 4-3. Comparative experiment between KAI1 knock-down and KAI1 overexpressed EML cells

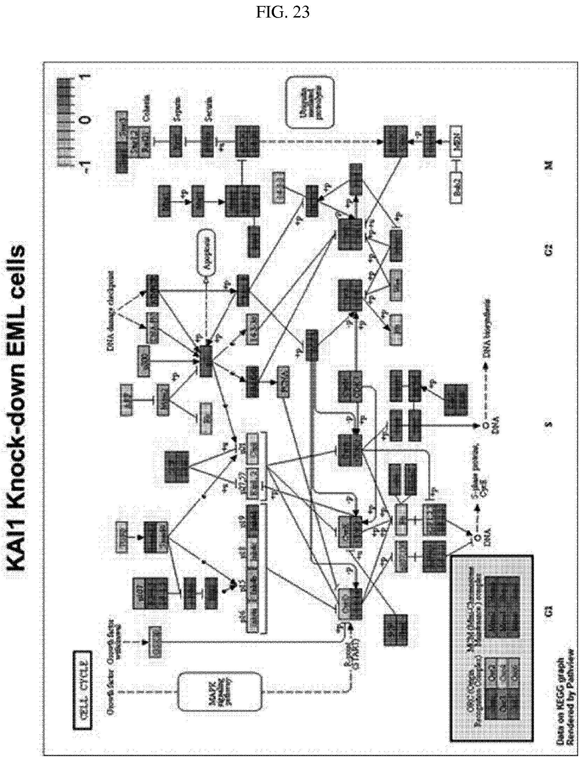

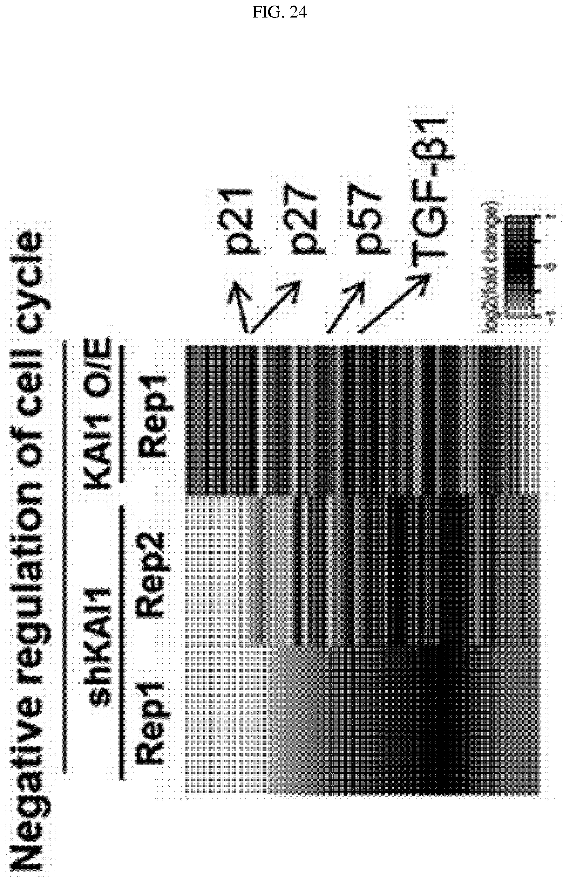

First, the KAI1 knock-down and KAI1 overexpressed EML cells obtained in Example 4-2 were subjected to RNA sequencing analysis. As a result, it can be confirmed that a CDK inhibitor was decreased and upstream TGF-β thereof was decreased in KAI1 knock-down EML cells, as shown in FIG. 23. In addition, it can be confirmed that the number of genes in the negative cell cycle were reduced in the KAI1 knock-down EML cells, whereas the number of genes in the negative cell cycle were increased in KAI1 overexpression EML cells, as shown in FIG. 24.

Subsequently, the increase/decrease in TGF-β1 and TGFR2 was confirmed using RT-PCR and Western blotting. Primer information used herein is shown in Table 5 below.

**[Table 5]**

| Type | Primer sequence | |
|---|---|---|
| TGF-β1 | FW (Forward) | 5'-CGGACTACTATGCTAAAGAGGTCAC-3' (SEQ ID NO: 26) |
| | RV (Reverse) | 5'-GAGTTTGTTATCTTTGCTGTCACAAG-3' (SEQ ID NO: 27) |
| TGFβR2 | FW (Forward) | 5'-CATCTTCTACTGCTACCGTGTCC-3' (SEQ ID NO: 28) |
| | RV (Reverse) | 5'-ATGTTCTCATGCTTCAGGTTGAT-3' (SEQ ID NO: 29) |

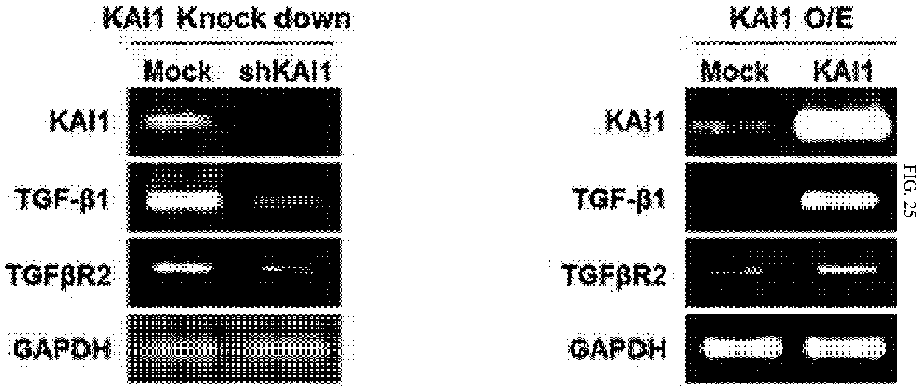

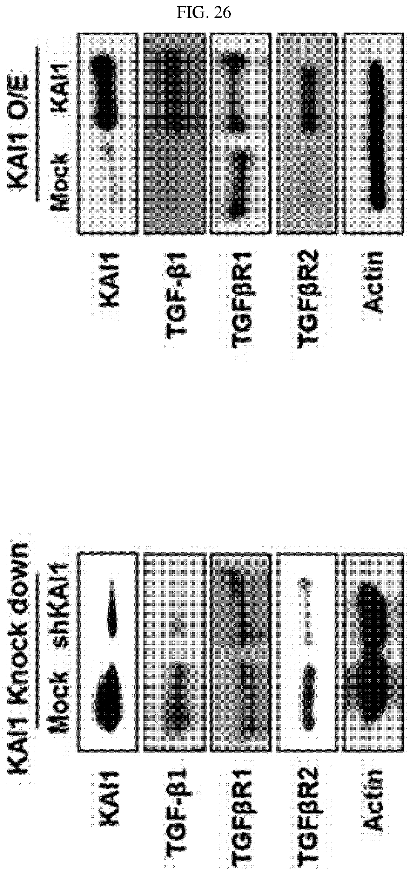

As shown in FIGS. 25 and 26, it can be confirmed that TGF-β1 and TGFβR2 expression was reduced in KAI1 knock-down EML cells, whereas TGF-β1 and TGFβR2 expression was increased in KAI1 Over-expressed EML cells.

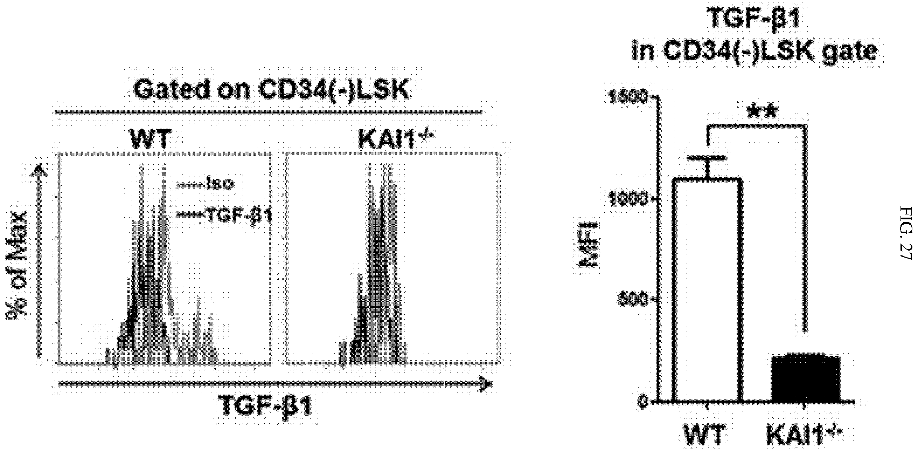

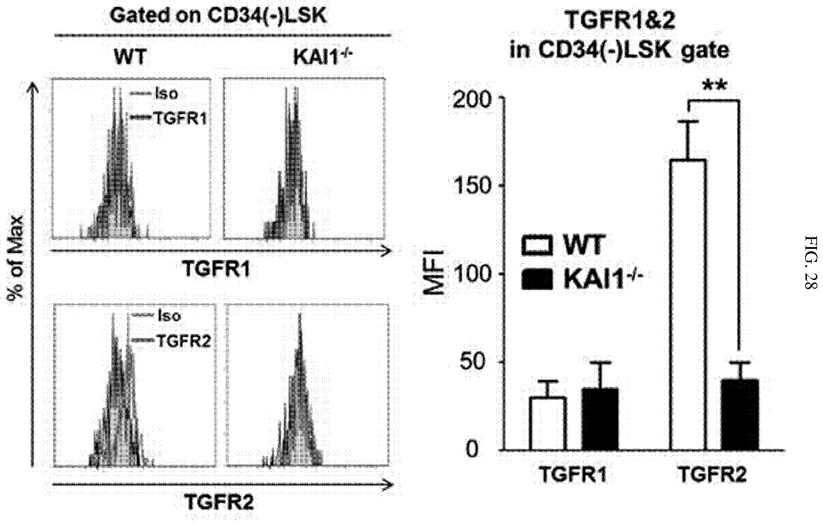

Subsequently, BM cells were obtained from actual femurs and tibias of WT and KAI1 K/O mice, and therefrom peripheral blood mononuclear cells (PBMCs) were isolated using histopaque-1083 (Sigma-Aldrich) for FACS analysis. As a result of comparing TGF-β1 and TGFR2 positive cells in LT-HSCs, which are Flt3-CD34-LSK cells, it can be confirmed that the TGF-β1 and TGFR2 positive cells were reduced in the KAI1 K/O mice, as shown in FIGS. 27 and 28.

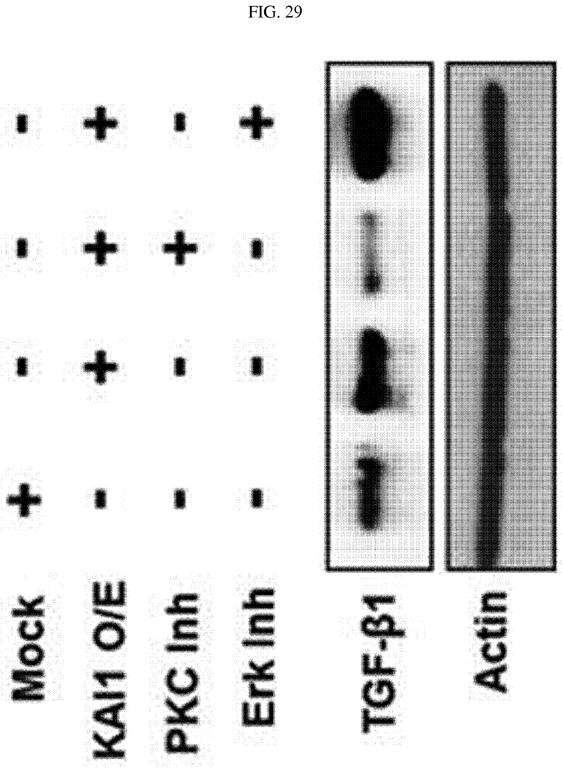

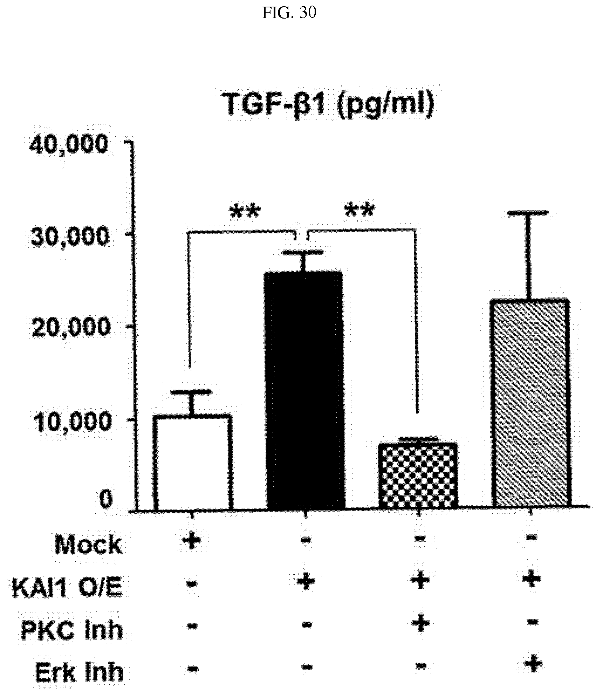

Afterwards, since TGF-β1 has been conventionally known to be increased by PKC and Erk pathways, KAI1 overexpressed EML cells were treated with their inhibitors to find a link between increasing TGF-β1 expression and KAI1, and TGF-β1 expression and secretion were confirmed using Western blotting. Consequently, as shown in FIGS. 29 and 30, it can be confirmed that KAI1 increased the TGF-β1 expression and secretion via PKC, not Erk.

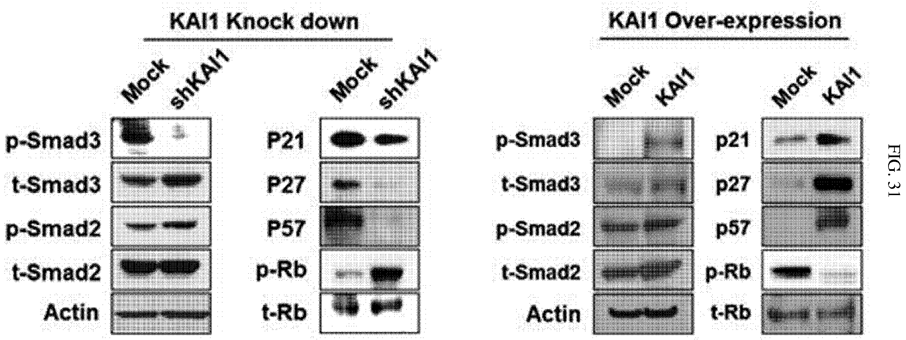

Then, in KAI1 knock-down and KAI1 overexpressed EML cells, Smad2 and Smand3 phosphorylation as downstream pathways of TGF-β1/TGFR and the increase/decrease in expression of CDK inhibitors such as p21, p27, and p57 were compared using Western blotting. As a result, as shown in FIG. 31, it can be confirmed that the p21, p27 and p57 as well as Smad3 phosphorylation were reduced in the KAI1 knock-down cells. On the other hand, the Rb phosphorylation was increased. However, it can be confirmed that the opposite results were attained in the overexpression of KAI1.

In addition, it can be determined that the p21, p27 and p57 increased by the KAI1 overexpression EML cells were decreased again in the TGFR inhibitor-treated group by treatment of the KAI1 overexpression EML cells with a TGFR inhibitor, confirming whether the CDK inhibitors were increased by TGF-β1. In addition, it can be confirmed that the result was obtained using even a neutralizing antibody of TGF-β1.

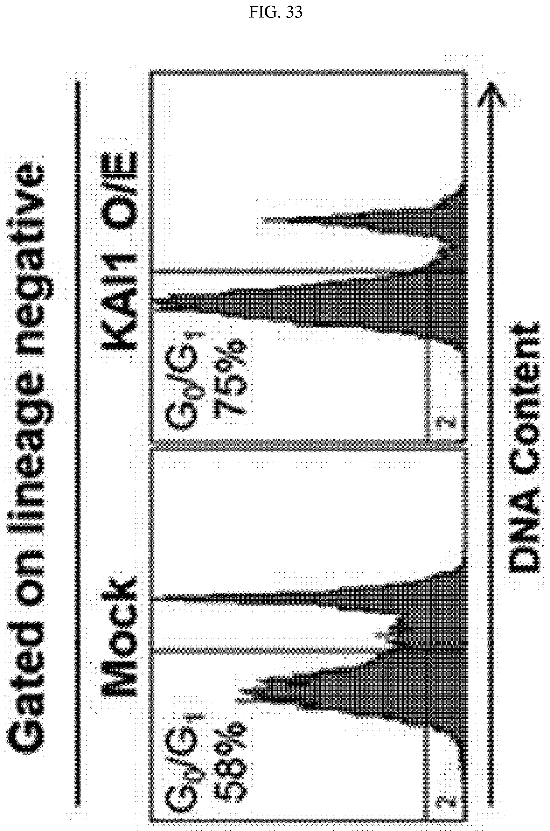

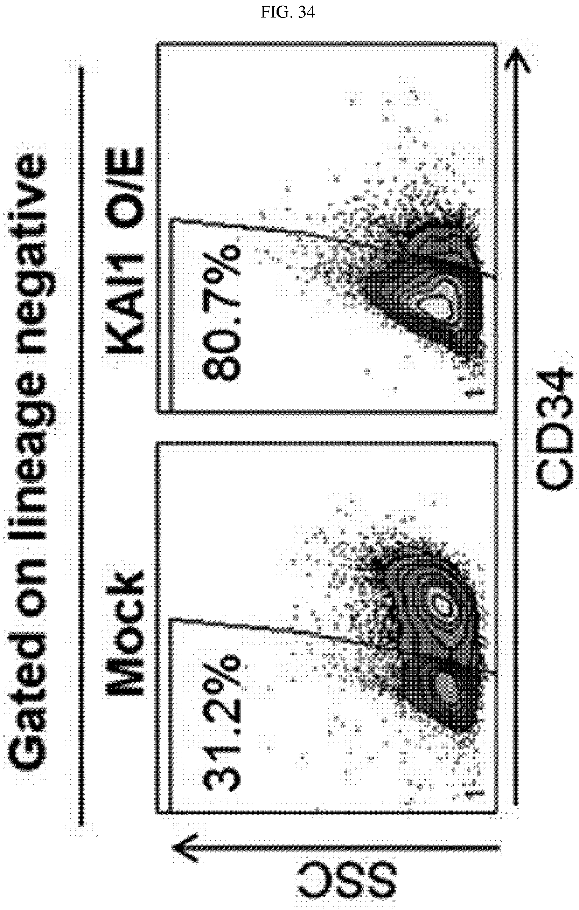

Moreover, to confirm whether the change in CDK inhibitor by the KAI1 overexpression actually affected a cell cycle, FACS was carried out on Mock/, KAI1 O/E EML to compare cell cycles. As a result, as shown in FIGS. 33 and 34, it can be confirmed that number of G0/G1 phase positive cells was increased (58% vs 75%) in KAI1 O/E EML, and a Lin- a Lin-CD34- population was increased more than CD34+ population known to activate cell cycling in EML cells.

### Example 5. Confirmation of role of KAI1(CD82) in LT-HSCs when BM was reconstructed after ablation

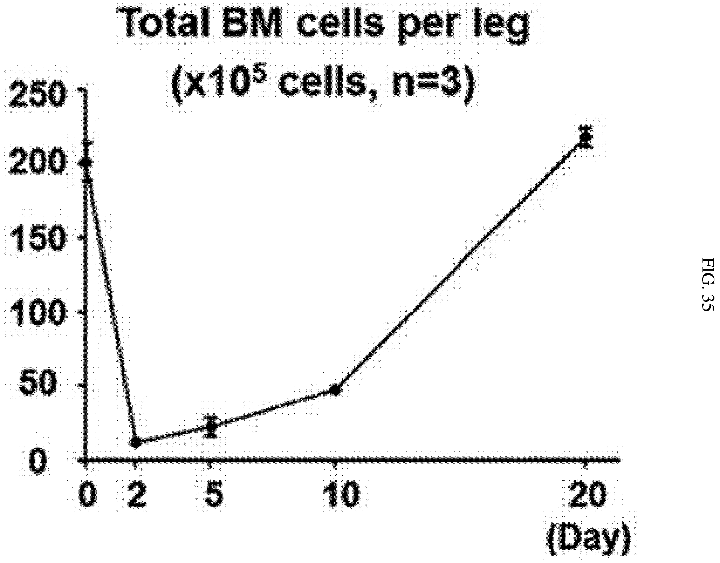

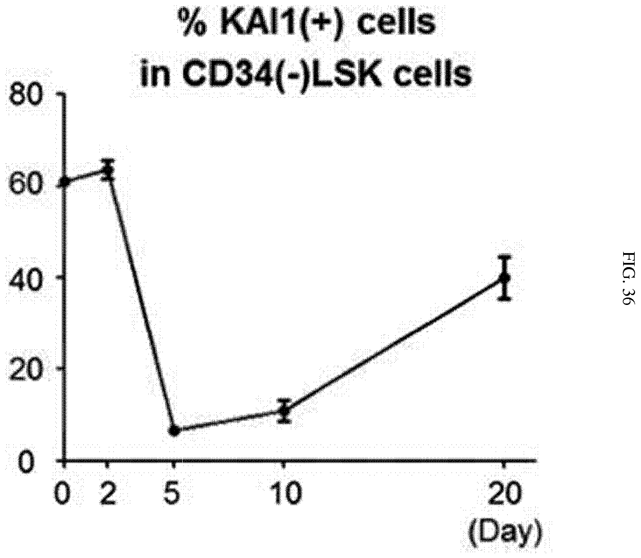

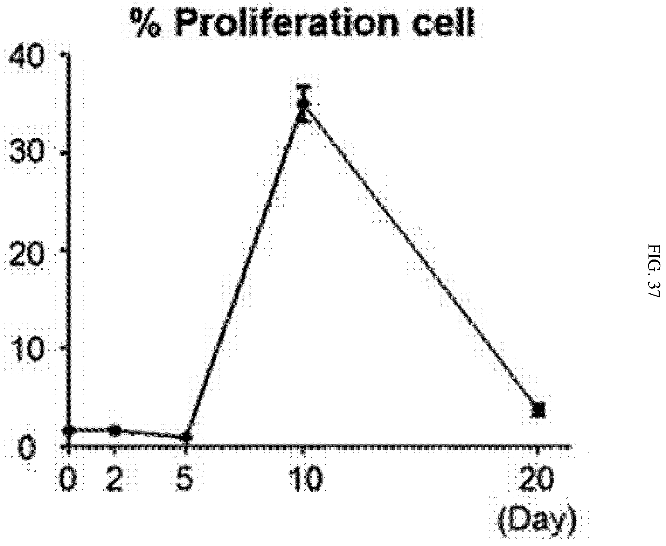

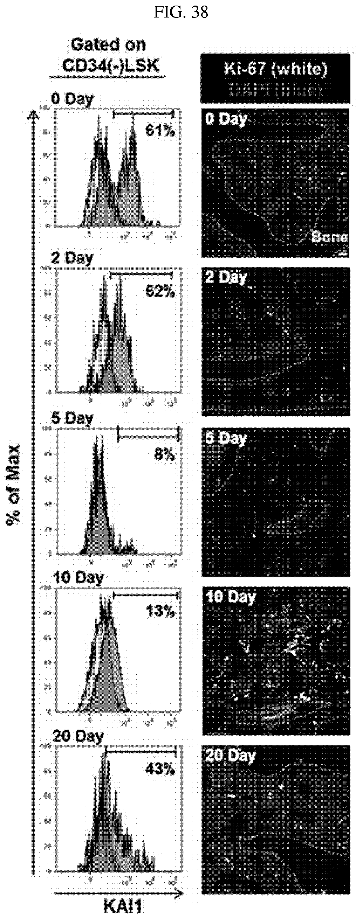

To confirm the role of KAI1 in BM reconstruction and maintenance of quiescent LT-HSCs after ablative intervention, 5-FU was injected into WT mice exposed to sub-lethal irradiation, and then continuous changes in KAIl-positive LT HSCs and proliferating blood cells in BM were confirmed. As a result, as shown in FIG. 35, it can be confirmed that since 5-FU induces apoptosis of actively cycling progenitors or blood cells, the number of cells in the BM was rapidly decreased. In addition, as shown in FIGS. 36 to 38, it can be confirmed that to provide new cells in response to the decrease in cells in BM due to the 5-FU treatment, KAI1 expression in LT-HSCs started to decrease on the second day, went to the lowest level on the 5^{th} day, and then recovered until the second week. Moreover, it can be confirmed that the number of cells starting to differentiate from BM was increased starting from the 5^{th} day due to the decrease in LT-HSC KAII expression.

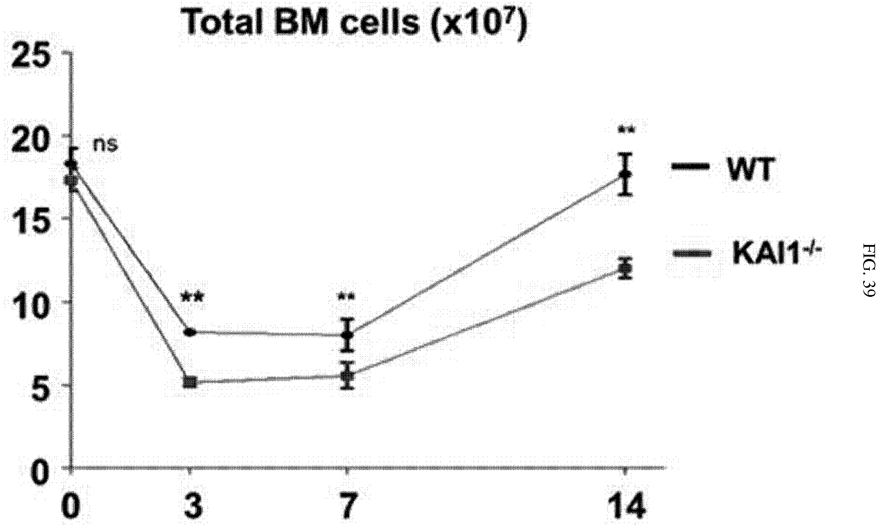

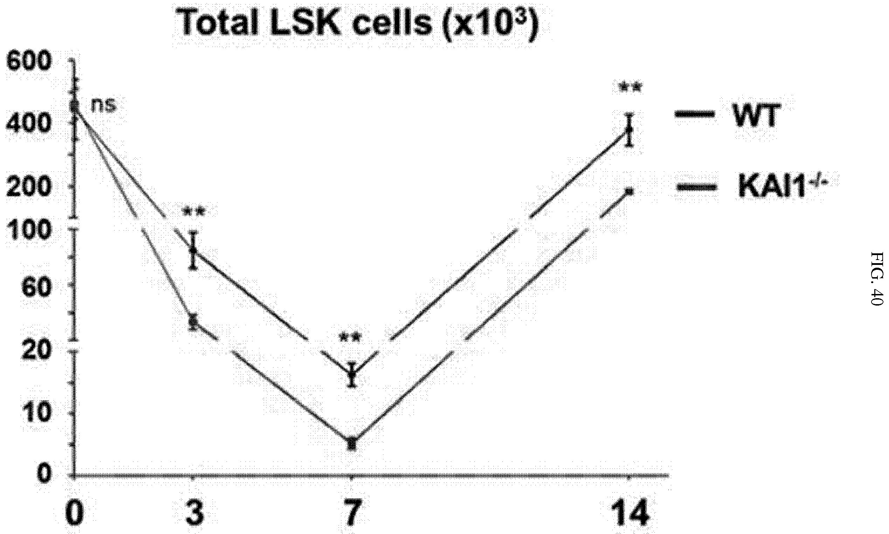

Furthermore, to confirm that KAI1 is important in LT-HSCs for recovering hematopoietic stem cells after the ablation of BM cells, total BM and LSK cell recovery in KAI1^{-/-} and WT mice after sub-lethal irradiation were compared. As a result, as shown in FIGS. 39 and 40, it can be confirmed that the recovery of total BM and LSK cells was lower in the KAI1^{-/-} mice than in WT.

Therethrough, it can be seen that the decrease in KAI1 in LT-HSCs was significant in BM reconstruction after ablation.

### Example 6. Confirmation of role of KAI1(CD82) in LT-HSCs and long term reconstitution after BM cell transplantation

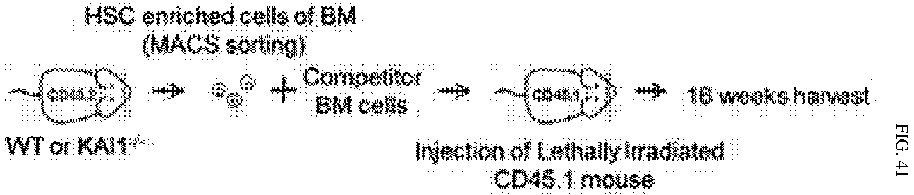

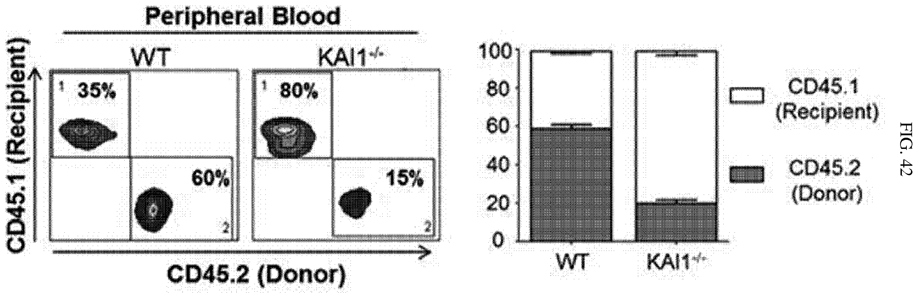

First, to determine if KAI1 affects long term repopulating capacity of HSCs, a competitive BM transplantation (BMT) experiment was carried out, and the process of the experiment is schematically shown in FIG. 41. That is, HSPC-enriched 1x10⁵ Lin(-) BM cells (CD45.2, WT) + 5x10⁵ competitor cells (CD45.1) or HSPC-enriched 1x10⁵ Lin(-) BM cells (CD45.2, KAI1^{-/-} mice) + 5x10⁵ competitor cells (CD45.1) were transplanted into lethally irradiated recipient mice (CD45.1), and after 16 weeks, BM cells were harvested for FACS analysis. Consequently, as shown in FIG. 42, it can be confirmed that KAI1^{-/-} cells exhibited a lower repopulating capability than WT cells.

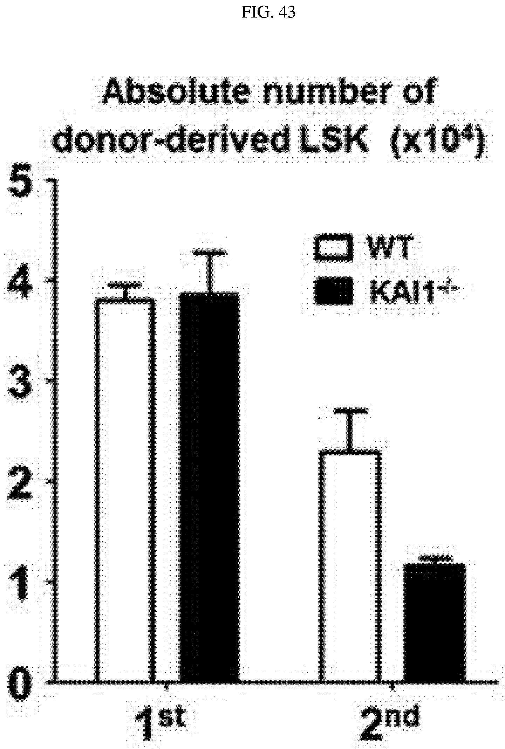

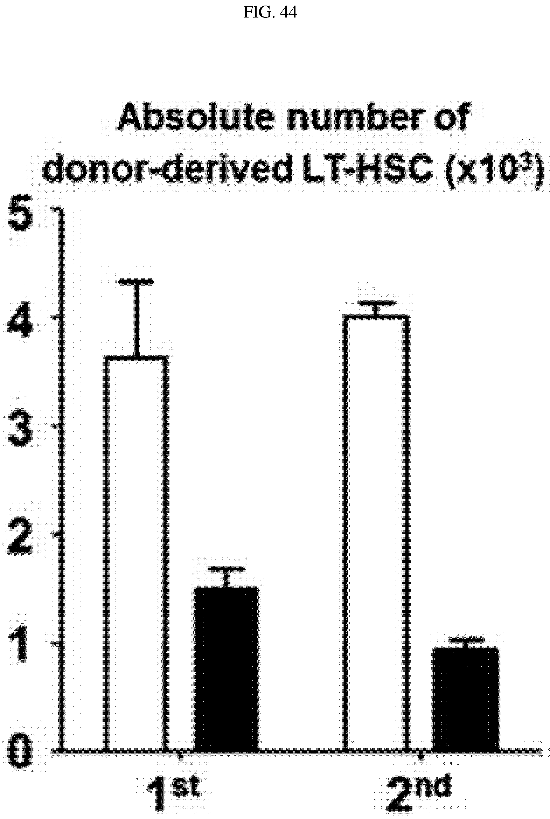

Subsequently, through serial BMT, the number of LSK cells and LT-HSCs at the second BMT were compared between BM cells of mice receiving HSPCs of the KAI1^{-/-} mice and BM cells of mice receiving HSPCs of WT mice. As a result, as shown in FIGS. 43 and 44, it can be confirmed that the number of LSK cells and LT-HSCs were lower in the BM cells of mice receiving HSPCs of the KAI1^{-/-} mice than in those of the mice receiving HSPCs of WT mice.

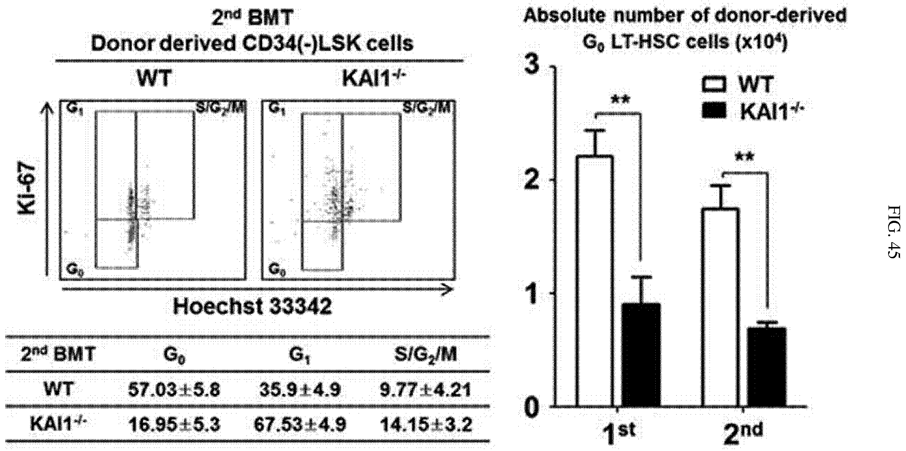

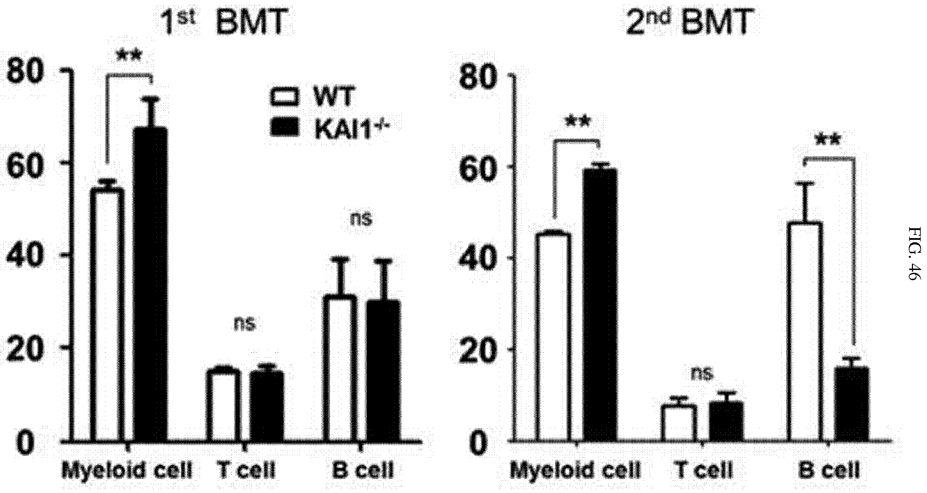

Afterwards, cell cycles in CD34- LSK cells of the mice receiving either KAI1^{-/-} mouse- or WT-derived HSPCs were compared. As a result, as shown in FIG. 45, it can be confirmed that the number and degree of G0 phase LT-HSCs were higher in the mice receiving the WT-derived HSPCs. In addition, as shown in FIG. 46, it can be confirmed that a differentiation direction of LT-HSCs went towards a myeloid lineage in the mice receiving the KAI1^{-/-} mouse-derived HSPCs.

### Example 7. Identification of KAI1 activity-associated ligand

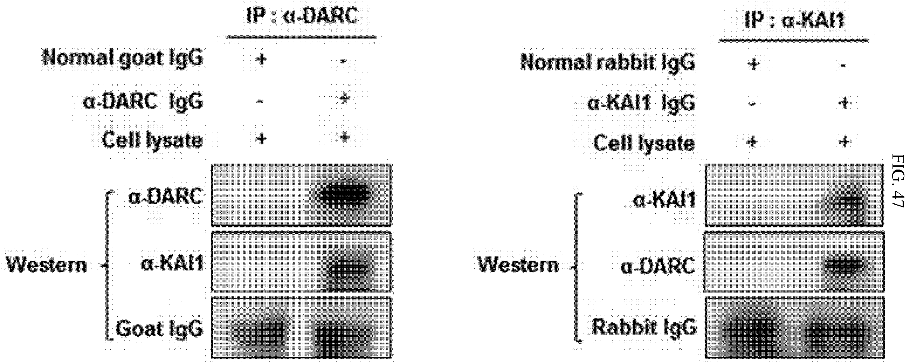

First, to find a ligand increasing KAI1 activity, immunoprecipitation on EML cells was carried out using a KAI1 antibody, and then Western blotting was carried out using a Duffy antigen receptor for chemokines (DARC). As a result, as shown in FIG. 47, it can be confirmed that KAI1 was able to interact with DARC. The same result was obtained from the immunoprecipitation (IP) of DARC and Western blotting of KAI1.

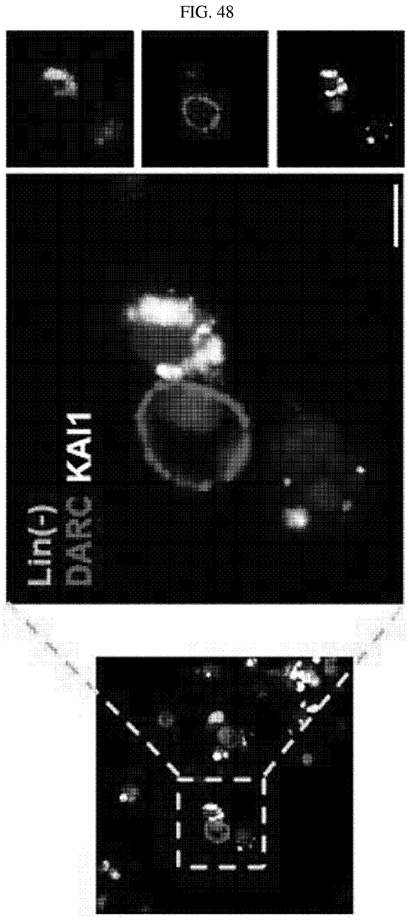

Subsequently, to show *in vitro* interaction between KAI1 and DARC, F4/80 positive cells were sorted from the bone of a C57 mouse using a MACS technique and seeded on a dish, and then an HSC enriched population of a C57 mouse, that is, Lin(-) cells of the C57 mouse, was sorted using a MACS technique and then tagged with CFSE. These two types of cells were co-cultured and then identified by staining with KAI1 and DARC. Therethrough, as shown in FIG. 48, it can be confirmed that KAI1 derived from HSCs and DARC derived from macrophages interacted with each other.

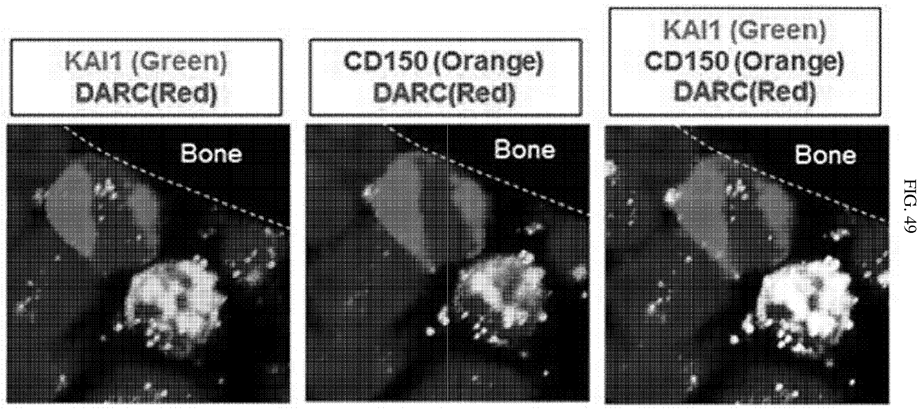

Afterwards, to confirm that KAI1 and DARC actually interacted with each other in vivo, a femur of the C57 mouse was fixed and decalcificated, followed by preparation of a paraffin block. After preparing sections, the resulting sections were subjected to deparaffinization and retrieval, and then fluorescence-stained using an antibody. As a result of staining with CD150 as a HSC marker, and as a result of KAI1 and DARC, it can be confirmed that KAI1(+)LT-HSC and DARC interacted with each other, as shown in FIG. 49A,.

In addition, a femur of a Tie2-GFP mouse expressing GFp only in cells expressing Tie2 was treated using the above-described method for preparing a paraffin block, preparing sections, and following with deparaffinization and retrieval for fluorescence staining. Here, Tie2 was LT-HSCs. As a result, as shown in FIG. 49B, it can be confirmed that KAI1(+)LT-HSC and DARC actually interacted with each other. From the above results, it can be seen that KAI1 derived from LT-HSC interacted with DARC.

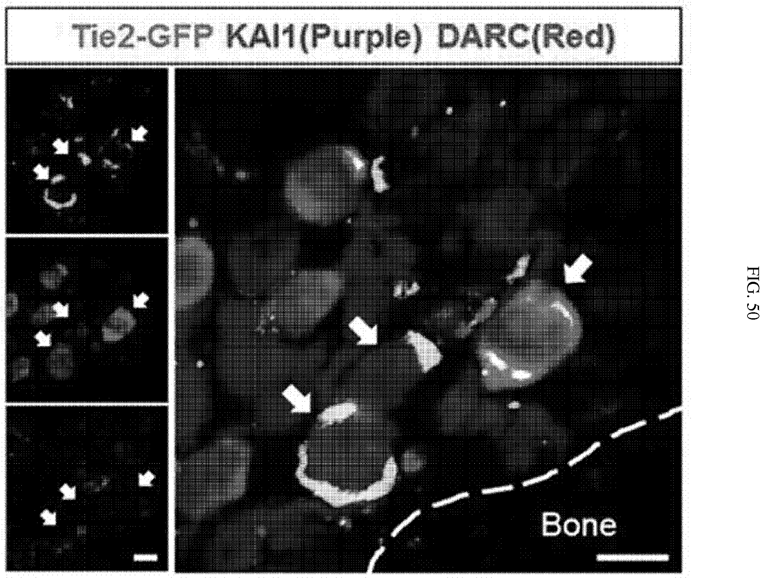

Then, to identify DARC(+) cells in BM, DARC expression was confirmed in endothelial cells, stromal cells, and monocytes/macrophages known to support HSCs, particularly in BM. Endothelial cells were identified from CD45(-)Ter119(-)CD31(+)PDGFRa(-), stromal cells were identified from CD45(-)Ter119(-)CD31(-)PDGFRa(+), and finally macrophages were identified from F4/80(+). As a result, as shown in FIG. 50, it can be confirmed that the endothelial cells and the stromal cells were mostly DARC negative cells, and the macrophages were mostly DARC positive cells.

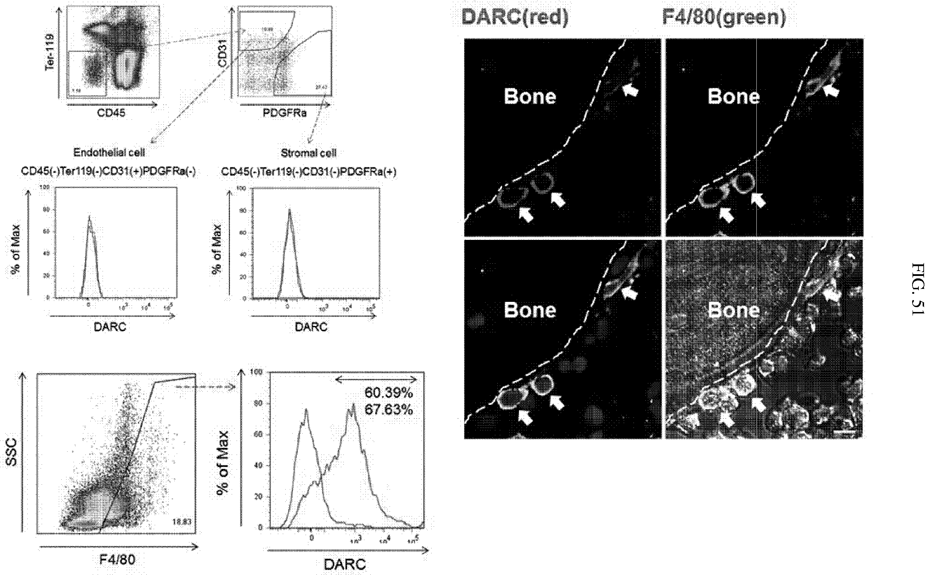

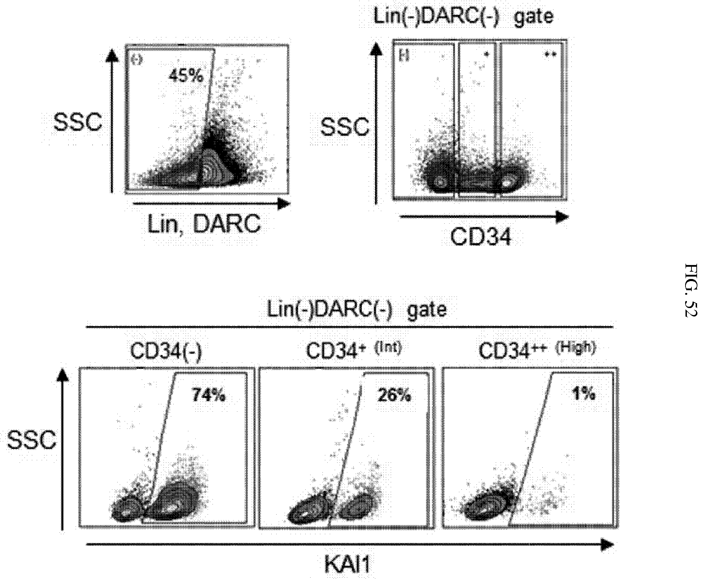

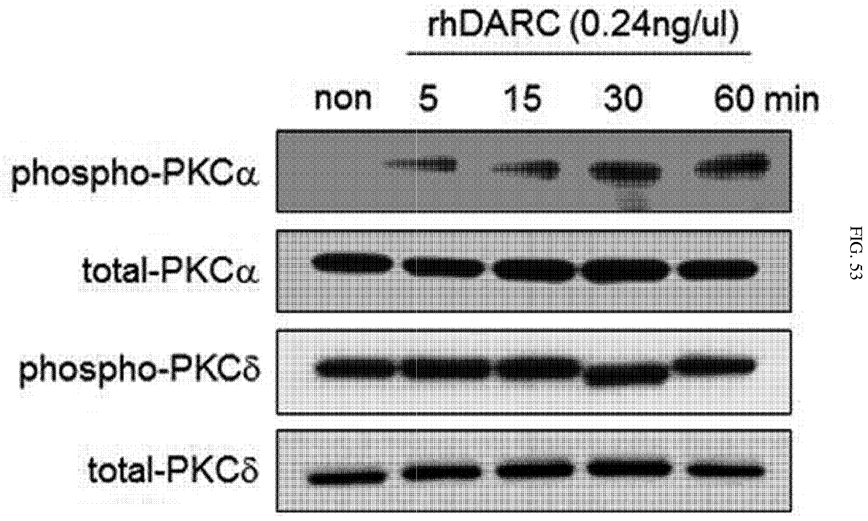

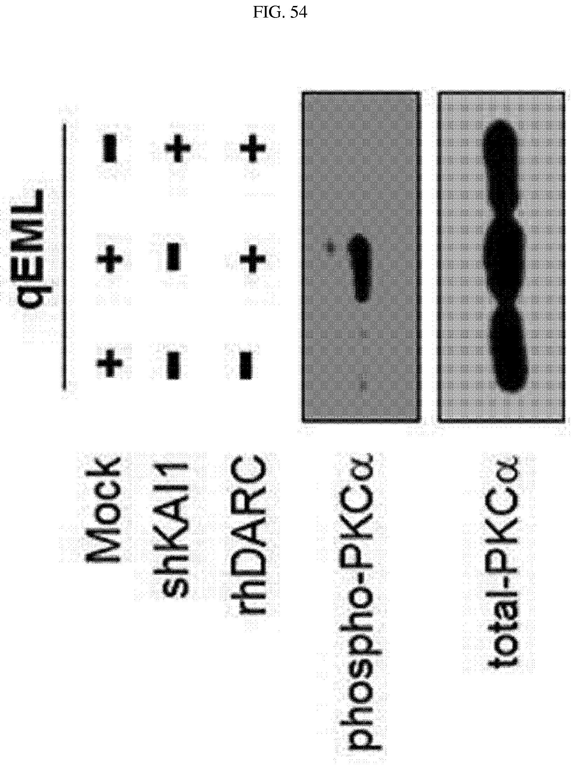

Meanwhile, in Lin(-)CD34(-), known as a quiescent cell population of EML cells, the percentage of KAI1 was approximately 80%. However, among the cell cycle-active population, Lin(-)CD34(+) cells exhibited a percentage of KAI1 of approximately 1%, which were mostly KAI1 negative cells (refer to FIG. 51). Here, to see an increase in KAI1 activity by recombinant DARC, Lin(-)DARC(-)CD34(-) cells, most of which were KAI1(+) EML cells, were sorted using a MACS technique. Specifically, EML cells were incubated with a lineage cocktail, DARC, and a CD34 primary antibody for 30 minutes at 4 °C, and then washed three times. The resultant cells were treated and incubated with a secondary antibody suitable for the primary antibody for 30 minutes at 4 °C and washed three times, thereby obtaining a Lin(-)DARC(-)CD34(-) quiescent EML cell population (qEML) using a magnetic column. The cells obtained thereby were subjected to starvation for 1 to 3 hours in an almost stem cell factor (FSC)-free, 1% FBS-containing IMDM medium for 3 hours, and then treated with rhDARC (recombinant human DARC) to confirm PKC phosphorylation by KAI1 activity. As a result, as shown in FIG. 52, it can be confirmed that PKCa activity was increased by rhDARC. From the result, it was seen that KAI1 can be activated by rhDARC. DARC has approximately 76% homology with a mouse and a human, and the rhDARC acts on a mouse. In addition, to see if rhDARC increases PKCa phosphorylation using KAI1, EML cells were transfected with a Mock shRNA lentivirus and a KAI1 shRNA lentivirus [KAI1 knock-down], and then Lin(-)CD34(-)DARC(-) quiescent EML cells were isolated and treated with rhDARC. As a result, as shown in FIG. 53, it was confirmed through KAI1 that rhDARC increased PKCa phosphorylation. Afterwards, to confirm that G0 phase was actually maintained in a qEML cell cycle by rhDARC or co-culture of monocytes in the qEML cells from which the previously mentioned Lin(-)CD34(-)DARC(-) EML cells were sorted, the cell cycle was analyzed. More specifically, a cell cycle was analyzed by co-culturing Mock raw 264.7 cells, DARC knock-down raw 264.7 cells, and finally, DARC knock-down raw 264.7 cells with qEML cells, and then incubating the resulting cells for 2 days after pretreating with rhDARC. Therethrough, as shown in FIG. 54, it can be confirmed that, when qEML cells were co-cultured with Raw 264.7 cells, G0 cells were maintained at a high level of approximately 61%, and when co-cultured with DARC Knock-down Raw 264.7 cells, G0 phase was reduced. Here, it was confirmed that the effects were reversed by treatment with rhDARC. Consequently, it was confirmed through mono/macrophage DARC that quiescence was maintained due to KAI1 of the qEML cells.

### Example 8. Confirmation of mechanism of regulating KAI1 expression in KAI1(+)LT-HSC

KAI1(+)LT-HSC had a resistance to stress, mobilization, and emergency niches, and in a subsequent recovery process, KAI1 expression was reduced, proliferated, or differentiated in LT-HSC. Therefore, the KAI1 expression was increased or decreased by mechanism of the LT-HSC.

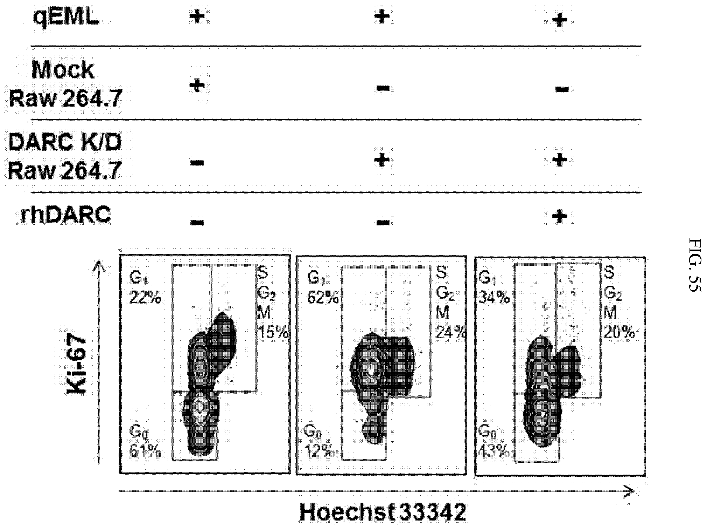

First, EML cells were mixed with various types of cells. Lin(-)CD34(-) cells were sorted using MACS, and cultured with a stem cell factor (SCF) to identify KAI1(+) cells, and therethrough, as shown in FIG. 55, it was confirmed that the KAI1(+) cells were reduced to 12% within two days. In addition, when the KAI1(+) cells were identified after co-culture with a mouse macrophage cell line, that is, Raw 264.7 cells, as shown in FIG. 55, it can be confirmed that KAI1-expressed cells in quiescent EML cells were maintained at a level of 83%, similar to that after MACS sorting, through the co-culture with the Raw 264.7 cells. From the above result, it can be seen that KAI1 expression in LT-HSC was maintained by macrophages, which have a direct effect and a paracrine effect.

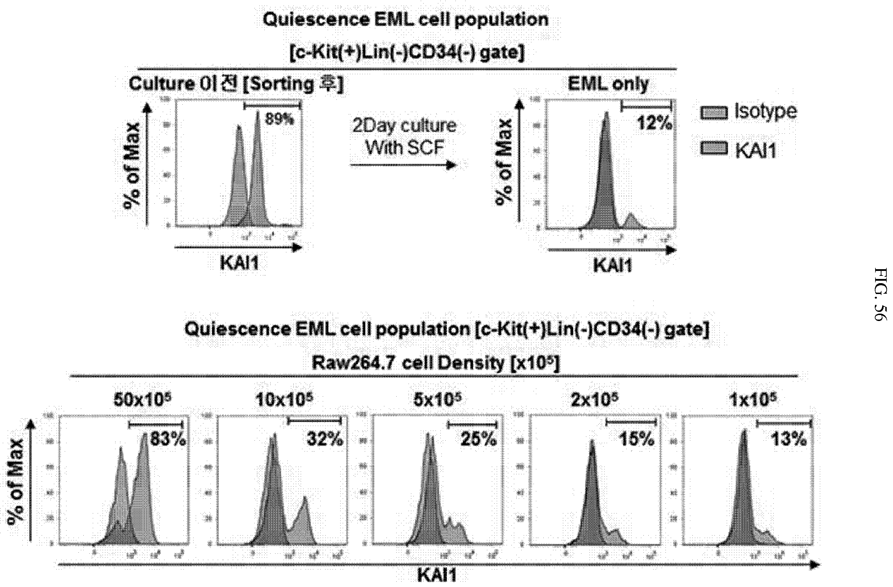

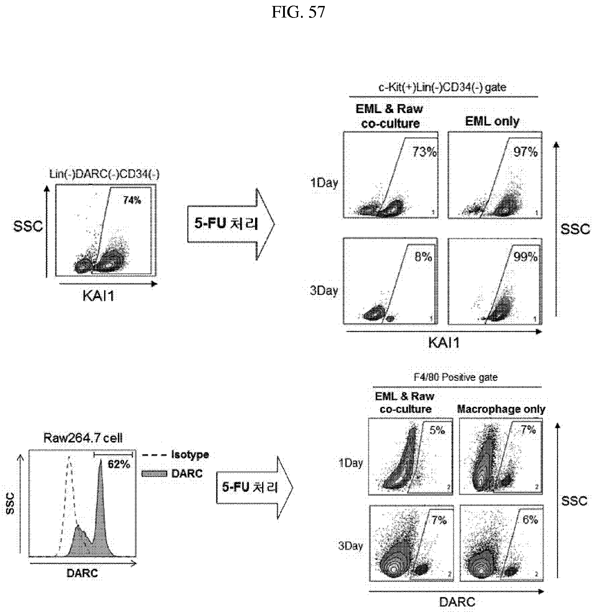

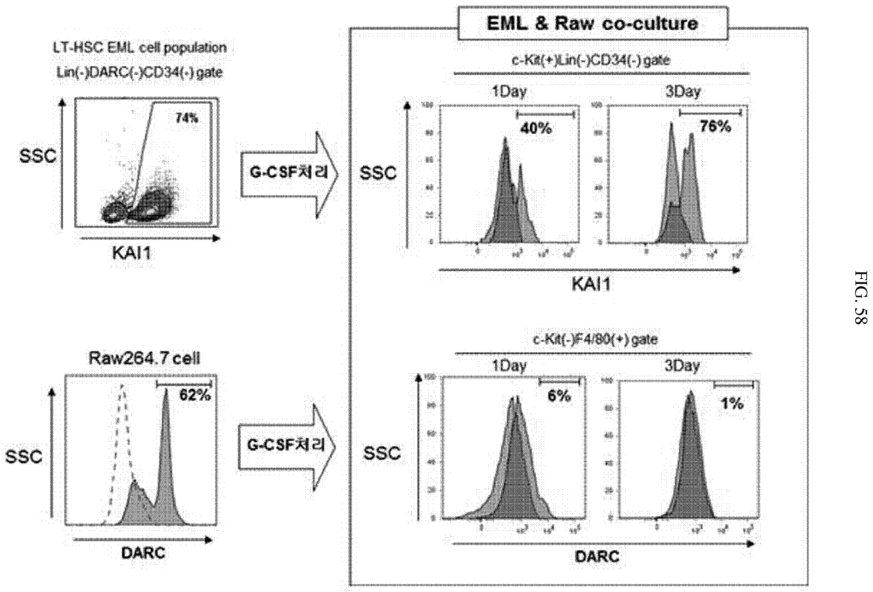

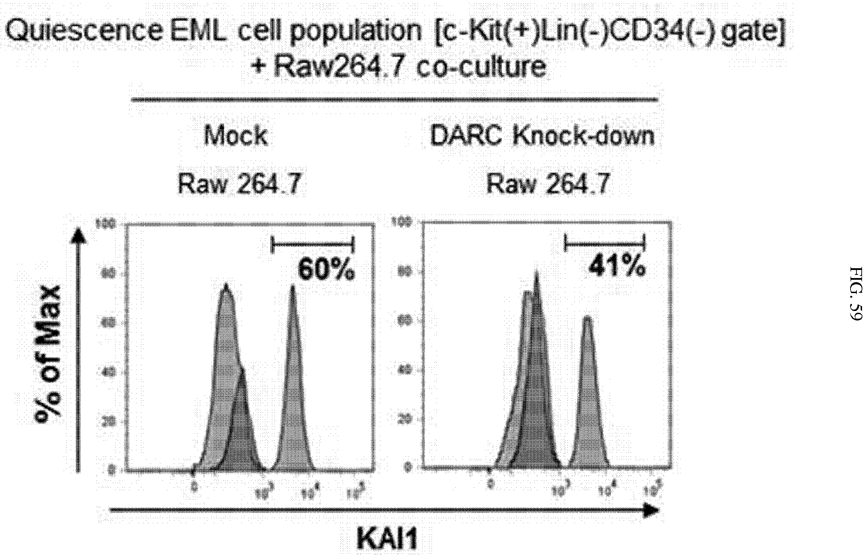

Then, when DARC expression in the Raw 264.7 cells was confirmed at a stress or mobilization niche such as G-CSF or 5-FU, as shown in FIGS. 56 and 57, it was confirmed that DARC expression was decreased at the stress or mobilization niche such as G-CSF or 5-FU, and KAI1 of LT-HSC was also reduced. From the result, it can be seen that the reduction in KAI1 of LT-HSC was caused by a direct or indirect effect of DARC. Afterwards, after the DARC expression in Raw 264.7 cells was reduced using shRNA, quiescent EML cells sorted in the same manner as described above from total EML cells and co-cultured with Mock Raw 264.7 and DARC Knock-down Raw264.7 cells under the condition of SCF addition. As a result, as shown in FIG. 58, it can be confirmed that KAI1 expression in the quiescent EML cells was reduced by co-culture with the DARC Knock-down Raw 264.7 cells. In addition, to see a paracrine effect, Mock and DARC Knock-down Raw 264.7 supernatants were obtained and cultured with EML cells. Consequently, as shown in FIG. 59, it can be confirmed that KAI1 expression was maintained even by a paracrine effect, and KAI1 expression in the quiescent EML cells was reduced in a supernatant obtained from the DARC Knock-down Raw 264.7 cells.

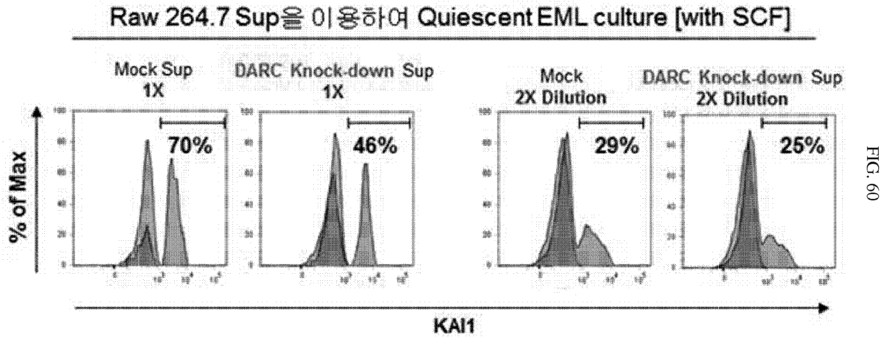

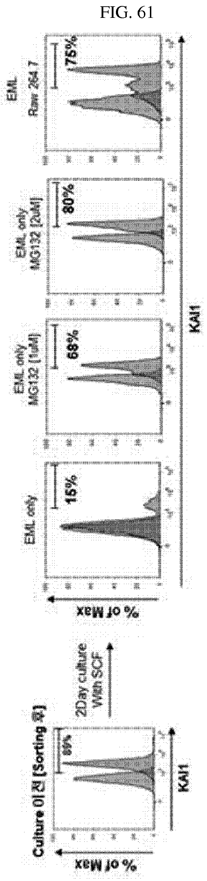

Moreover, since KAI1 was reduced by two processes such as ubiquitination and endocytosis, qEML cells were sorted from EML cells and cultured with SCF. Therethrough, as shown in FIG. 60, it can be confirmed that under the condition of addition of a ubiquitin degradation inhibitor such as MG-132, KAI1 expression was not reduced. In addition, the qEML was cultured with SCF, and after 8 hours, KAI1 was immunoprecipitated, and mono/poly-ubiquitin was detected using Western blotting, thereby confirming ubiquitinated KAI1, as shown in FIG. 61.

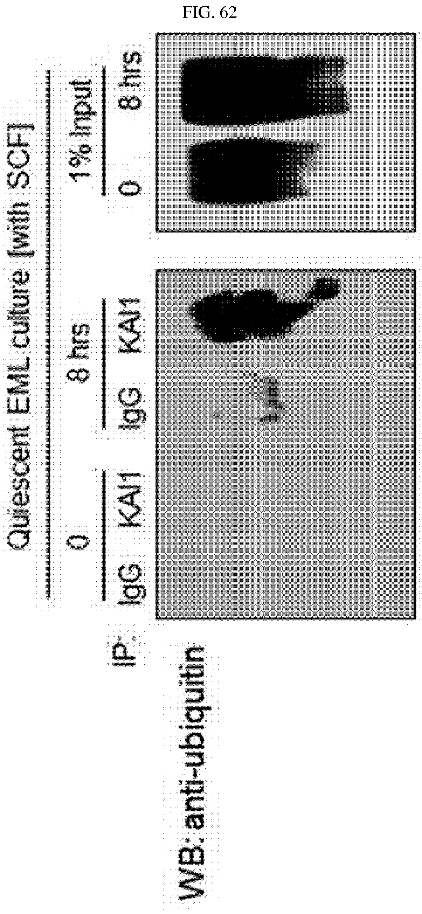

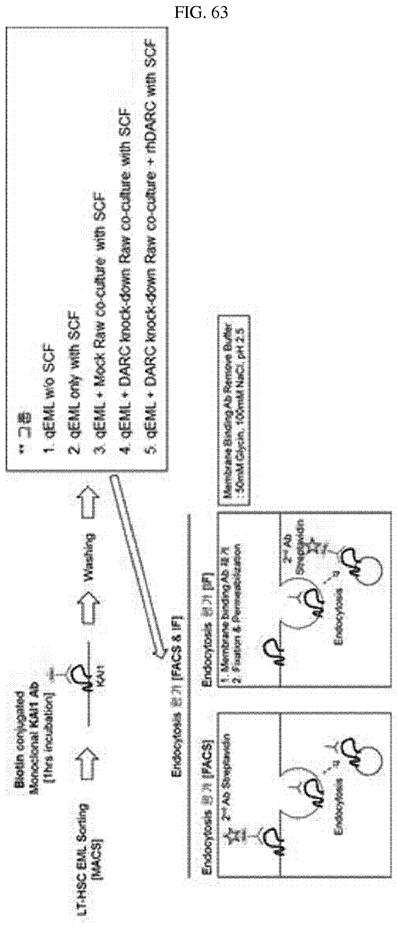

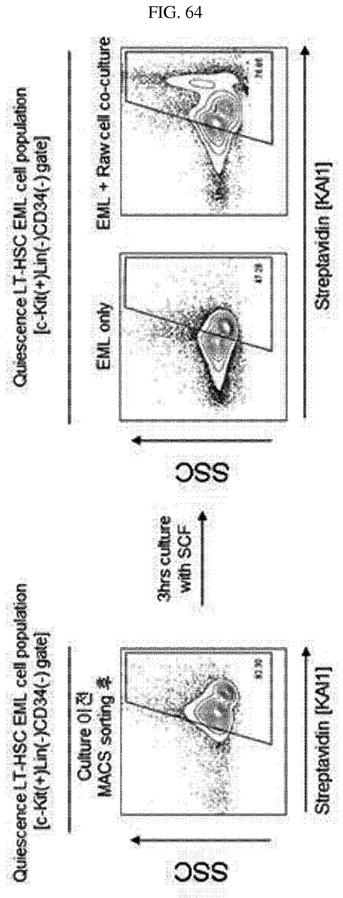

Furthermore, to evaluate endocytosis, qEML cells were obtained from EML using MACS in the same manner as described above, incubated with a biotin-conjugated KAI1 antibody at 4 °C for 1 hour, and then washed to remove a non-bound antibody. Afterwards, to identify qEML only, qEML + Mock Raw 264.7 cells, qEML + DARC Knock-down Raw 264.7, qEML + DARC Knock-down Raw 264.7 + rhDARC, and finally, to confirm that the endocytosis was caused by ubiquitination, qEML cells were pretreated with MG-132. In addition, all groups were treated with SCF, cultured for 3 hours, and then had a membrane-binding antibody removed using an acidic buffer (50 mM glycine, 100 mM NaCl, pH 2.5). Moreover, to identify endocytic KAIl-biotin conjugation, a streptavidin-555 secondary antibody was used after fixation/permeabilization. As a result, as shown in FIGS. 62 to 64, [SCF] KAI1 was ubiquitinated by a growth factor when only qEML was cultured, resulting in endocytosis, and therefore KAI1 was reduced. However, when co-culturing with a monocyte/macrophage first, it can be confirmed that KAI1 endocytosis was not reduced by DARC.

### Example 9. Confirmation of role of KAI1(CD82) in maintaining quiescence of human LT-HSC

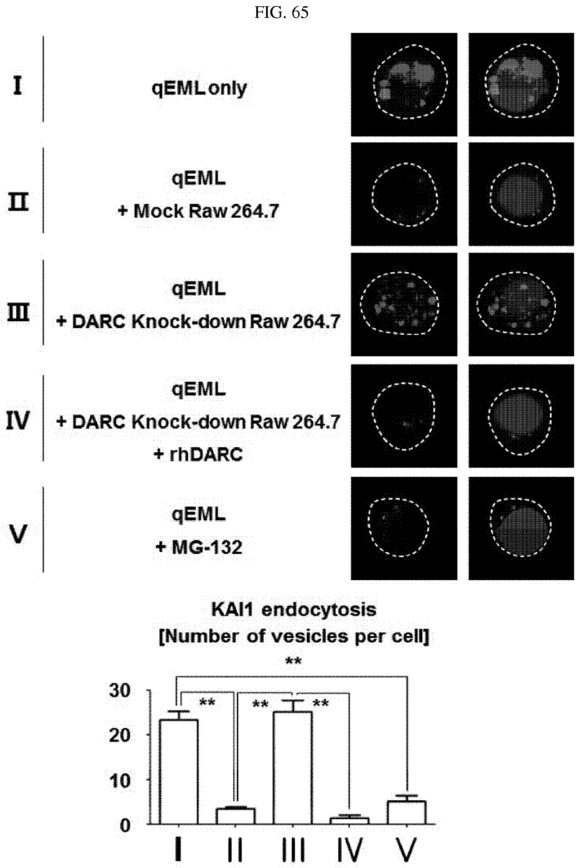

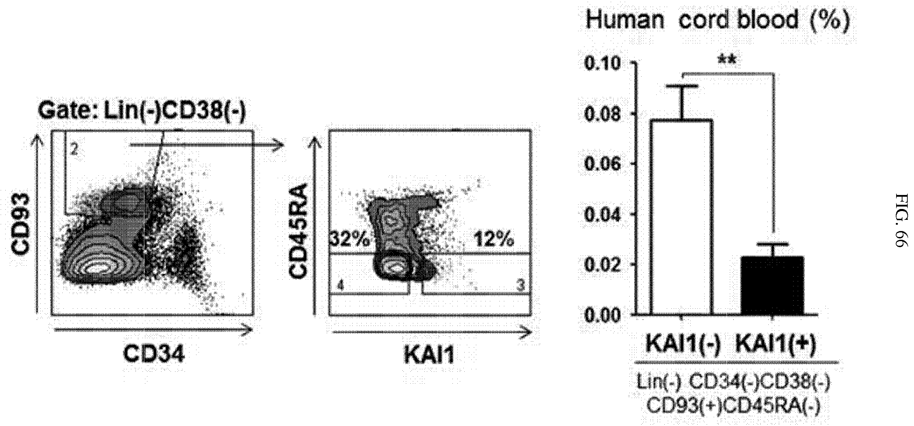

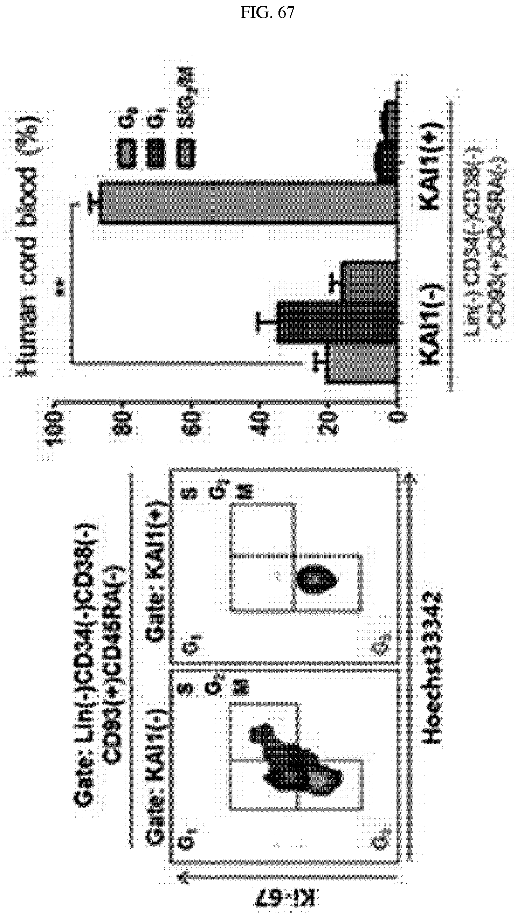

To confirm if the experimental result showing that KAI1 expression is critical for maintaining the quiescence of mouse LT-HSC is also applicable to humans, an experiment was carried out using human umbilical cord blood from a blood vessel of an umbilical cord. The umbilical cord blood was collected with a heparin-coated syringe under the consent of a donor and delivered. Monocytes contained in the blood were collected using histopaque 1077. These were stained with a LIN, CD38, CD34, CD93, or CD45RA antibody, and analyzed by FACS. As a result, as shown in FIGS. 65 and 66, it can be confirmed that from the LIN(-)CD38(-)CD34(-)CD93(+)CD45RA(-) population, KAI1(+) LT-HSCs derived from human LT-HSCs accounted for approximately 25%, and unlike a KAI1(-) population, most of the KAI1(+) LT-HSCs were at G0 phase. From the result, it can be seen that the human LT-HSCs expressed KAI1, and KAI1-expressed human LT-HSCs were quiescent. In addition, first MNC was obtained first from the human umbilical cord blood and Lin(+)CD235(+)CD235(+) cells were removed using MACS, and then KAI1(+)LT-HSC and KAI1(-)LT-HSC were sorted using FACS. Then, following 3-hour starvation, the cells were treated with rhDARC for 30 minutes, and subjected to fixation/permeabilization and fluorescence staining to confirm PKCα phosphorylation. As a result, as shown in FIG. 67, it can be confirmed that, in humans, PKCα phosphorylation was increased in the KAI1(+)LT-HSC by rhDARC.

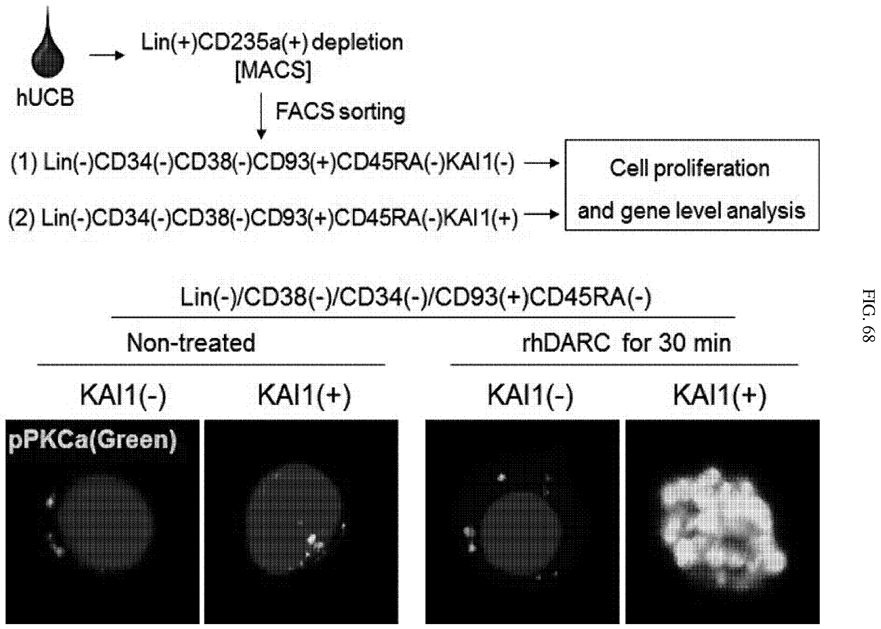

Afterwards, as an experiment to confirm if quiescence of KAI1(+)LT-HSC was increased by rhDARC, MNC was obtained from human umbilical cord blood by the same method as described above, Lin(+)CD235(+)DARC(+) cells were removed, and following 3-hour starvation, the cells were treated with rhDARC for 2 days, and then a cell cycle was analyzed. As a result, as shown in FIG. 68, it can be confirmed that the number of cells in G0 phase was increased in KAI1(+)LT-HSC due to rhDARC.

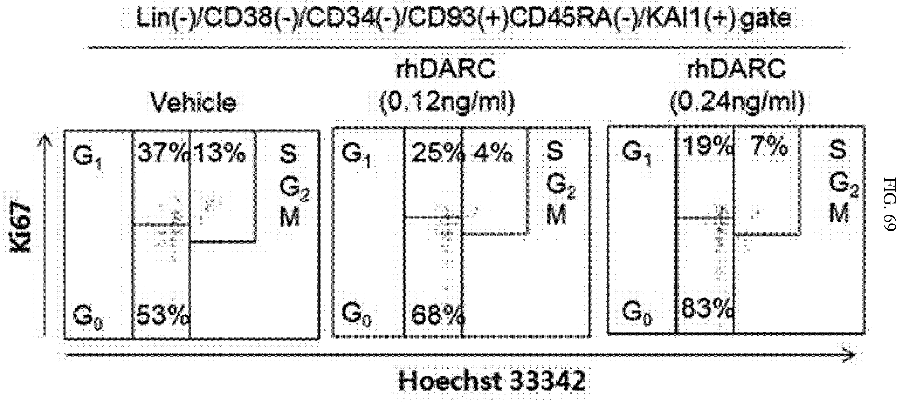

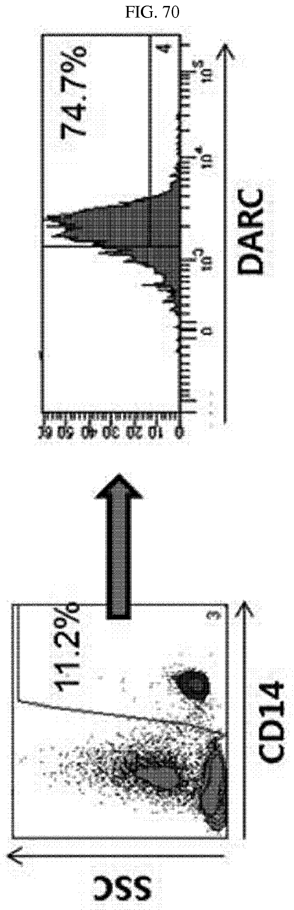

Subsequently, according to the experiment subjected for a human carried out in the same manner as described above, as shown in FIG. 69, it can be confirmed that in cord blood, most of CD14 positive cells as a monocyte/macrophage marker were DARC positive cells. Finally, to confirm if KAI1 expression in LT-HSC was also regulated by monocytes in humans, Lin(-)CD235(-)DARC(-) cells were obtained from umbilical cord blood by the same method as described above and some of those cells were isolated as monocytes [un-touched monocytes] using MACS. According to a [SCF, Flt3-ligand, TPO, IL-3] HSC-only culture obtained using a growth factor, it can be confirmed that, as shown in FIG. 70, KAI1 was reduced, but KAI1 expression was maintained by the co-culture with a monocyte or rhDARC without reduction.

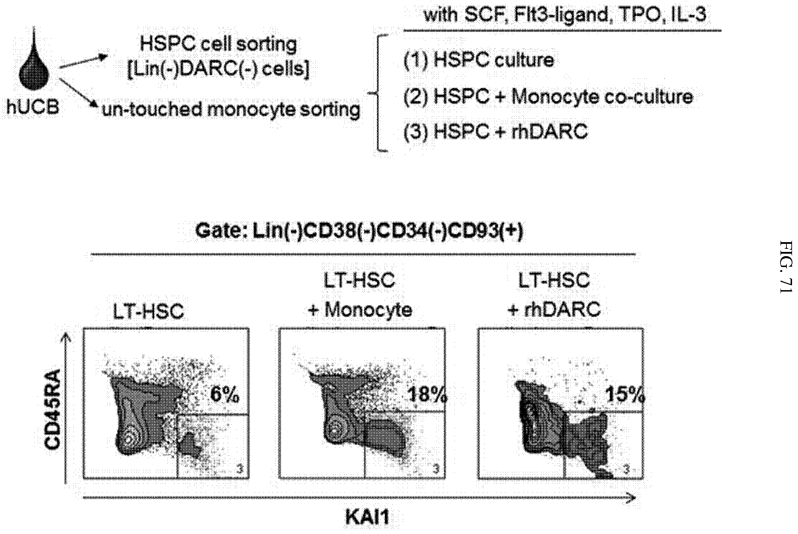

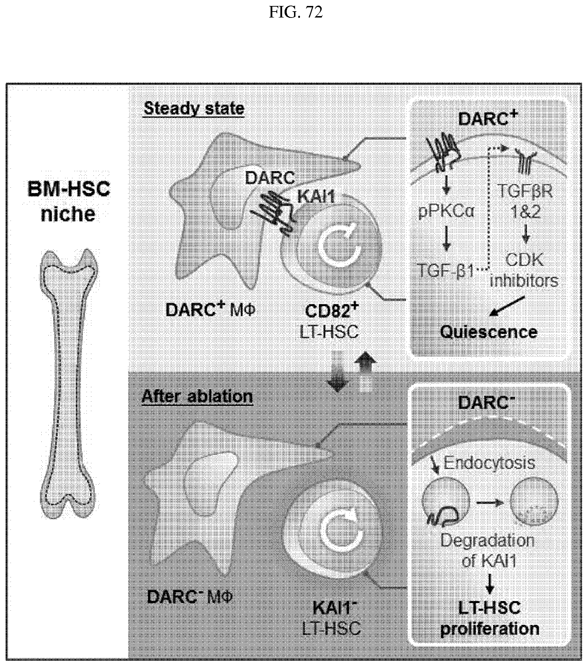

The above-mentioned results are summarized and shown in FIG. 71. As shown in FIG. 71, first, KAI1 of LT-HSC was stimulated by DARC of monocytes/macrophages in homeostasis [increase in KAI1 phosphorylation] to phosphorylate PKCa, increase TGFb, and increase CDK inhibitors such as p21, p27, and p57 via Smad2/3 through TGFR1&2, thereby reducing phospho-Rb and resulting in maintaining the quiescence of the LT-HSC. Second, in a situation involving an emergency/mobilization/stressed niche such as 5-FU or G-CSF, due to the reduction in expression of the DARC of the monocytes/macrophages or the number of monocytes/macrophages, a KAI1/DARC complex was broken, and thereby the KAI1 of the LT-HSC was ubiquitinated and subjected to endocytosis by a neighboring growth factor. Therefore, the cell cycle and differentiation of KAI1(-) LT-HSC were improved, resulting in bone marrow regeneration. DARC(+)monocytes/macrophages were increased again by the bone marrow regeneration. Particularly, the KA1I(-)LT-HSC was able to be proliferated and differentiate into the monocyte/macrophage. As a result, the KAI1 of the LT-HSC was increased again, and maintained homeostasis.

### Sequence Listing

<210> 1
   <211> 267
   <212> PRT
   <213> Homo sapiens CD82
<400> 1
<210> 2
   <211> 266
   <212> PRT
   <213> Mus musculus CD82
<400> 2
   Met Gly Ala Gly Cys Val Lys Val Thr Lys Tyr Phe Leu Phe Leu Phe
   1 5 10 15
   Asn Leu Leu Phe Phe Ile Leu Gly Ala Val Ile Leu Gly Phe Gly Val
   20 25 30
   Trp Ile Leu Ala Asp Lys Asn Ser Phe Ile Ser Val Leu Gln Thr Ser
   35 40 45
   Ser Ser Ser Leu Gln Val Gly Ala Tyr Val Phe Ile Gly Val Gly Ala
   50 55 60
   Ile Thr Ile Val Met Gly Phe Leu Gly Cys Ile Gly Ala Val Asn Glu
   65 70 75 80
   Val Arg Cys Leu Leu Gly Leu Tyr Phe Val Phe Leu Leu Leu Ile Leu
   85 90 95
   Ile Ala Gln Val Thr Val Gly Val Leu Phe Tyr Phe Asn Ala Asp Lys
   100 105 110
   Leu Lys Lys Glu Met Gly Asn Thr Val Met Asp Ile Ile Arg Asn Tyr
   115 120 125
   Thr Ala Asn Ala Thr Ser Ser Arg Glu Glu Ala Trp Asp Tyr Val Gln
   130 135 140
   Ala Gln Val Lys Cys Cys Gly Trp Val Ser His Tyr Asn Trp Thr Glu
   145 150 155 160
   Asn Glu Glu Leu Met Gly Phe Thr Lys Thr Thr Tyr Pro Cys Ser Cys
   165 170 175
   Glu Lys Ile Lys Glu Glu Asp Asn Gln Leu Ile Val Lys Lys Gly Phe
   180 185 190
   Cys Glu Ala Asp Asn Ser Thr Val Ser Glu Asn Asn Pro Glu Asp Trp
   195 200 205
   Pro Val Asn Thr Glu Gly Cys Met Glu Lys Ala Gln Ala Trp Leu Gln
   210 215 220
   Glu Asn Phe Gly Ile Leu Leu Gly Val Cys Ala Gly Val Ala Val Ile
   225 230 235 240
   Glu Leu Leu Gly Leu Phe Leu Ser Ile Cys Leu Cys Arg Tyr Ile His
   245 250 255
   Ser Glu Asp Tyr Ser Lys Val Pro Lys Tyr
   260 265
<210> 3
   <211> 338
   <212> PRT
   <213> Homo sapiens DARC
<400> 3
   Met Ala Ser Ser Gly Tyr Val Leu Gln Ala Glu Leu Ser Pro Ser Thr
   1 5 10 15
   Glu Asn Ser Ser Gln Leu Asp Phe Glu Asp Val Trp Asn Ser Ser Tyr
   20 25 30
   Gly Val Asn Asp Ser Phe Pro Asp Gly Asp Tyr Gly Ala Asn Leu Glu
   35 40 45
   Ala Ala Ala Pro Cys His Ser Cys Asn Leu Leu Asp Asp Ser Ala Leu
   50 55 60
   Pro Phe Phe Ile Leu Thr Ser Val Leu Gly Ile Leu Ala Ser Ser Thr
   65 70 75 80
   Val Leu Phe Met Leu Phe Arg Pro Leu Phe Arg Trp Gln Leu Cys Pro
   85 90 95
   Gly Trp Pro Val Leu Ala Gln Leu Ala Val Gly Ser Ala Leu Phe Ser
   100 105 110
   Ile Val Val Pro Val Leu Ala Pro Gly Leu Gly Ser Thr Arg Ser Ser
   115 120 125
   Ala Leu Cys Ser Leu Gly Tyr Cys Val Trp Tyr Gly Ser Ala Phe Ala
   130 135 140
   Gln Ala Leu Leu Leu Gly Cys His Ala Ser Leu Gly His Arg Leu Gly
   145 150 155 160
   Ala Gly Gln Val Pro Gly Leu Thr Leu Gly Leu Thr Val Gly Ile Trp
   165 170 175
   Gly Val Ala Ala Leu Leu Thr Leu Pro Val Thr Leu Ala Ser Gly Ala
   180 185 190
   Ser Gly Gly Leu Cys Thr Leu Ile Tyr Ser Thr Glu Leu Lys Ala Leu
   195 200 205
   Gln Ala Thr His Thr Val Ala Cys Leu Ala Ile Phe Val Leu Leu Pro
   210 215 220
   Leu Gly Leu Phe Gly Ala Lys Gly Leu Lys Lys Ala Leu Gly Met Gly
   225 230 235 240
   Pro Gly Pro Trp Met Asn Ile Leu Trp Ala Trp Phe Ile Phe Trp Trp
   245 250 255
   Pro His Gly Val Val Leu Gly Leu Asp Phe Leu Val Arg Ser Lys Leu
   260 265 270
   Leu Leu Leu Ser Thr Cys Leu Ala Gln Gln Ala Leu Asp Leu Leu Leu
   275 280 285
   Asn Leu Ala Glu Ala Leu Ala Ile Leu His Cys Val Ala Thr Pro Leu
   290 295 300
   Leu Leu Ala Leu Phe Cys His Gln Ala Thr Arg Thr Leu Leu Pro Ser
   305 310 315 320
   Leu Pro Leu Pro Glu Gly Trp Ser Ser His Leu Asp Thr Leu Gly Ser
   325 330 335
   Lys Ser
<210> 4
   <211> 334
   <212> PRT
   <213> Mus musculus DARC
<400> 4 325 330
<210> 5
   <211> 23
   <212> DNA
   <213> Artificial Sequence
<220>
   <223> CD9 forward primer
<400> 5
   agtgcatcaa atacctgctc ttc 23
<210> 6
   <211> 23
   <212> DNA
   <213> Artificial Sequence
<220>
   <223> CD9 reverse primer
<400> 6
   ctttaatcac ctcatccttg tgg 23
<210> 7
   <211> 23
   <212> DNA
   <213> Artificial Sequence
<220>
   <223> CD37 forward primer
<400> 7
   cttcgttttc aacctcttct tct 23
<210> 8
   <211> 23
   <212> DNA
   <213> Artificial Sequence
<220>
   <223> CD37 reverse primer
<400> 8
   aactgtgcat agtcccaact ctc 23
<210> 9
   <211> 23
   <212> DNA
   <213> Artificial Sequence
<220>
   <223> CD81 forward primer
<400> 9
   ttctacgtgg gcatctacat tct 23
<210> 10
   <211> 23
   <212> DNA
   <213> Artificial Sequence
<220>
   <223> CD81 reverse primer
<400> 10
   gctgttcctc agtatggtgg tag 23
<210> 11
   <211> 23
   <212> DNA
   <213> Artificial Sequence
<220>
   <223> CD151 forward primer
<400> 11
   tgcctcaagt acctgctctt tac 23
<210> 12
   <211> 23
   <212> DNA
   <213> Artificial Sequence
<220>
   <223> CD151 reverse primer
<400> 12
   ctgactggtg gtatctcttg acc 23
<210> 13
   <211> 24
   <212> DNA
   <213> Artificial Sequence
<220>
   <223> KAI1(CD82) forward primer type I
<400> 13
   cactacaact ggacagagaa cgag 24
<210> 14
   <211> 25
   <212> DNA
   <213> Artificial Sequence
<220>
   <223> KAI1(CD82) reverse primer type I
<400> 14
   tgtagtcttc agaatgaatg taccg 25
<210> 15
   <211> 20
   <212> DNA
   <213> Artificial Sequence
<220>
   <223> Primer A
<400> 15
   gggtccccta ggaaattcaa 20
<210> 16
<211> 25
   <212> DNA
   <213> Artificial Sequence
<220>
   <223> Primer B
<400> 16
   atgatgcaga tgttctctca gggtg 25
<210> 17
   <211> 22
   <212> DNA
   <213> Artificial Sequence
<220>
   <223> Primer C
<400> 17
   acaggggact caccctacaa gg 22
<210> 18
   <211> 23
   <212> DNA
   <213> Artificial Sequence
<220>
   <223> P21 forward primer
<400> 18
   gagaacggtg gaactttgac ttc 23
<210> 19
   <211> 24
   <212> DNA
   <213> Artificial Sequence
<220>
   <223> P21 reverse primer
<400> 19
   gtgatagaaa tctgtcaggc tggt 24
<210> 20
   <211> 22
   <212> DNA
   <213> Artificial Sequence
<220>
   <223> P27 forward primer
<400> 20
   catgaagaac taacccggga ct 22
<210> 21
   <211> 22
   <212> DNA
   <213> Artificial Sequence
<220>
   <223> P27 reverse primer
<400> 21
   cagagtttgc ctgagaccca at 22
<210> 22
   <211> 23
   <212> DNA
   <213> Artificial Sequence
<220>
   <223> P57 forward primer
<400> 22
   agatctgacc tcagacccaa ttc 23
<210> 23
   <211> 22
   <212> DNA
   <213> Artificial Sequence
<220>
   <223> P57 reverse primer
<400> 23
   gctcttgatt ctcgtcctgc tc 22
<210> 24
   <211> 18
   <212> DNA
   <213> Artificial Sequence
<220>
   <223> KAI1(CD82) forward primer type II
<400> 24
   gcctgggact acgtgcag 18
<210> 25
   <211> 21
   <212> DNA
   <213> Artificial Sequence
<220>
   <223> KAI1(CD82) reverse primer type II
<400> 25
   cctcgttctc tgtccagttg t 21
<210> 26
   <211> 25
   <212> DNA
   <213> Artificial Sequence
<220>
   <223> TGF-beta1 forward primer
<400> 26
   cggactacta tgctaaagag gtcac 25
<210> 27
   <211> 26
   <212> DNA
   <213> Artificial Sequence
<220>
   <223> TGF-beta1 reverse primer
<400> 27
   gagtttgtta tctttgctgt cacaag 26
<210> 28
   <211> 23
   <212> DNA
   <213> Artificial Sequence
<220>
   <223> TGF-beta R2 forward primer
<400> 28
   catcttctac tgctaccgtg tcc 23
<210> 29
   <211> 23
   <212> DNA
   <213> Artificial Sequence
<220>
   <223> TGF-beta R2 reverse primer
<400> 29
   atgttctcat gcttcaggtt gat 23
<110> SEOUL NATIONAL UNIVERSITY R&DB FOUNDATION
<120> KAI1 PROTEIN CONTROLING CELL CYCLE OF HEMATOPOIETIC STEM CELL, AND USES THEREOF
<130> MPCT16-024
<150> KR 10-2015-0063203
   <151> 2015-05-06
<160> 29
<170> KoPatentIn 3.0
<210> 1
   <211> 267
   <212> PRT
   <213> Homo sapiens CD82
<400> 1
<210> 2
   <211> 266
   <212> PRT
   <213> Mus musculus CD82
<400> 2
   Met Gly Ala Gly Cys Val Lys Val Thr Lys Tyr Phe Leu Phe Leu Phe
   1 5 10 15
   Asn Leu Leu Phe Phe Ile Leu Gly Ala Val Ile Leu Gly Phe Gly Val
   20 25 30
   Trp Ile Leu Ala Asp Lys Asn Ser Phe Ile Ser Val Leu Gln Thr Ser
   35 40 45
   Ser Ser Ser Leu Gln Val Gly Ala Tyr Val Phe Ile Gly Val Gly Ala
   50 55 60
   Ile Thr Ile Val Met Gly Phe Leu Gly Cys Ile Gly Ala Val Asn Glu
   65 70 75 80
   Val Arg Cys Leu Leu Gly Leu Tyr Phe Val Phe Leu Leu Leu Ile Leu
   85 90 95
   Ile Ala Gln Val Thr Val Gly Val Leu Phe Tyr Phe Asn Ala Asp Lys
   100 105 110
   Leu Lys Lys Glu Met Gly Asn Thr Val Met Asp Ile Ile Arg Asn Tyr
   115 120 125
   Thr Ala Asn Ala Thr Ser Ser Arg Glu Glu Ala Trp Asp Tyr Val Gln
   130 135 140
   Ala Gln Val Lys Cys Cys Gly Trp Val Ser His Tyr Asn Trp Thr Glu
   145 150 155 160
   Asn Glu Glu Leu Met Gly Phe Thr Lys Thr Thr Tyr Pro Cys Ser Cys
   165 170 175
   Glu Lys Ile Lys Glu Glu Asp Asn Gln Leu Ile Val Lys Lys Gly Phe
   180 185 190
   Cys Glu Ala Asp Asn Ser Thr Val Ser Glu Asn Asn Pro Glu Asp Trp
   195 200 205
   Pro Val Asn Thr Glu Gly Cys Met Glu Lys Ala Gln Ala Trp Leu Gln
   210 215 220
   Glu Asn Phe Gly Ile Leu Leu Gly Val Cys Ala Gly Val Ala Val Ile
   225 230 235 240
   Glu Leu Leu Gly Leu Phe Leu Ser Ile Cys Leu Cys Arg Tyr Ile His
   245 250 255
   Ser Glu Asp Tyr Ser Lys Val Pro Lys Tyr
   260 265
<210> 3
   <211> 338
   <212> PRT
   <213> Homo sapiens DARC
<400> 3
   Met Ala Ser Ser Gly Tyr Val Leu Gln Ala Glu Leu Ser Pro Ser Thr
   1               5                   10                  15
   Glu Asn Ser Ser Gln Leu Asp Phe Glu Asp Val Trp Asn Ser Ser Tyr
               20                  25                  30
   Gly Val Asn Asp Ser Phe Pro Asp Gly Asp Tyr Gly Ala Asn Leu Glu
           35                  40                  45
   Ala Ala Ala Pro Cys His Ser Cys Asn Leu Leu Asp Asp Ser Ala Leu
       50                  55                  60
   Pro Phe Phe Ile Leu Thr Ser Val Leu Gly Ile Leu Ala Ser Ser Thr
   65                  70                  75                  80
   Val Leu Phe Met Leu Phe Arg Pro Leu Phe Arg Trp Gln Leu Cys Pro
                   85                  90                  95
   Gly Trp Pro Val Leu Ala Gln Leu Ala Val Gly Ser Ala Leu Phe Ser
               100                 105                 110
   Ile Val Val Pro Val Leu Ala Pro Gly Leu Gly Ser Thr Arg Ser Ser
           115                 120                 125
   Ala Leu Cys Ser Leu Gly Tyr Cys Val Trp Tyr Gly Ser Ala Phe Ala
       130                 135                 140
   Gln Ala Leu Leu Leu Gly Cys His Ala Ser Leu Gly His Arg Leu Gly
   145                 150                 155                 160
   Ala Gly Gln Val Pro Gly Leu Thr Leu Gly Leu Thr Val Gly Ile Trp
                   165                 170                 175
   Gly Val Ala Ala Leu Leu Thr Leu Pro Val Thr Leu Ala Ser Gly Ala
               180                 185                 190
   Ser Gly Gly Leu Cys Thr Leu Ile Tyr Ser Thr Glu Leu Lys Ala Leu
           195                 200                 205
   Gln Ala Thr His Thr Val Ala Cys Leu Ala Ile Phe Val Leu Leu Pro
       210                 215                 220
   Leu Gly Leu Phe Gly Ala Lys Gly Leu Lys Lys Ala Leu Gly Met Gly
   225                 230                 235                 240
   Pro Gly Pro Trp Met Asn Ile Leu Trp Ala Trp Phe Ile Phe Trp Trp
                   245                 250                 255
   Pro His Gly Val Val Leu Gly Leu Asp Phe Leu Val Arg Ser Lys Leu
               260                 265                 270
   Leu Leu Leu Ser Thr Cys Leu Ala Gln Gln Ala Leu Asp Leu Leu Leu
           275                 280                 285
   Asn Leu Ala Glu Ala Leu Ala Ile Leu His Cys Val Ala Thr Pro Leu
       290                 295                 300
   Leu Leu Ala Leu Phe Cys His Gln Ala Thr Arg Thr Leu Leu Pro Ser
   305                 310                 315                 320
   Leu Pro Leu Pro Glu Gly Trp Ser Ser His Leu Asp Thr Leu Gly Ser
                   325                 330                 335
   Lys Ser
<210> 4
   <211> 334
   <212> PRT
   <213> Mus musculus DARC
<400> 4
   Met Gly Asn Cys Leu Tyr Pro Val Glu Asn Leu Ser Leu Asp Lys Asn
   1               5                   10                  15
   Gly Thr Gln Phe Thr Phe Asp Ser Trp Asn Tyr Ser Phe Glu Asp Asn
               20                  25                  30
   Tyr Ser Tyr Glu Leu Ser Ser Asp Tyr Ser Leu Thr Pro Ala Ala Pro
           35                  40                  45
   Cys Tyr Ser Cys Asn Leu Leu Gly Arg Ser Ser Leu Pro Phe Phe Met
       50                  55                  60
   Leu Thr Ser Val Leu Gly Met Leu Ala Ser Gly Gly Ile Leu Phe Ala
   65                  70                  75                  80
   Ile Leu Arg Pro Phe Phe His Trp Gln Ile Cys Pro Ser Trp Pro Ile
                   85                  90                  95
   Leu Ala Glu Leu Ala Val Gly Ser Ala Leu Phe Ser Ile Ala Val Pro
               100                 105                 110
   Ile Leu Ala Pro Gly Leu His Ser Ala His Ser Thr Ala Leu Cys Asn
           115                 120                 125
   Leu Gly Tyr Trp Val Trp Tyr Thr Ser Ala Phe Ala Gln Ala Leu Leu
       130                 135                 140
   Ile Gly Cys Tyr Ala Cys Leu Asn Pro Arg Leu Asn Ile Gly Gln Leu
   145                 150                 155                 160
   Arg Gly Phe Thr Leu Gly Leu Ser Val Gly Leu Trp Gly Ala Ala Ala
                   165                 170                 175
   Leu Leu Gly Leu Pro Val Ala Leu Ala Ser Asp Ala Tyr Asn Gly Phe
               180                 185                 190
   Cys Ala Phe Pro Ser Ser Arg Asp Met Glu Ala Leu Lys Tyr Met His
           195                 200                 205
   Tyr Ala Ile Cys Phe Thr Ile Phe Thr Val Leu Pro Pro Thr Leu Leu
       210                 215                 220
   Ala Ala Lys Gly Leu Lys Ile Ala Leu Ser Lys Gly Pro Gly Pro Trp
   225                 230                 235                 240
   Val Ser Val Leu Trp Ile Trp Phe Ile Phe Trp Trp Pro His Gly Met
                   245                 250                 255
   Val Leu Ile Phe Asp Ala Leu Val Arg Ser Lys Ile Val Leu Leu Tyr
               260                 265                 270
   Thr Cys Gln Ser Gln Lys Ile Leu Asp Ala Met Leu Asn Val Thr Glu
           275                 280                 285
   Ala Leu Ser Met Leu His Cys Val Ala Thr Pro Leu Leu Leu Ala Leu
       290                 295                 300
   Phe Cys His Gln Thr Thr Arg Arg Ser Leu Ser Ser Leu Ser Leu Pro
   305                 310                 315                 320
   Thr Arg Gln Ala Ser Gln Met Asp Ala Leu Ala Gly Lys Ser
                   325                 330
<210> 5
   <211> 23
   <212> DNA
   <213> Artificial Sequence
<220>
   <223> CD9 forward primer
<400> 5
   agtgcatcaa atacctgctc ttc 23
<210> 6
   <211> 23
   <212> DNA
   <213> Artificial Sequence
<220>
   <223> CD9 reverse primer
<400> 6
   ctttaatcac ctcatccttg tgg 23
<210> 7
   <211> 23
   <212> DNA
   <213> Artificial Sequence
<220>
   <223> CD37 forward primer
<400> 7
   cttcgttttc aacctcttct tct 23
<210> 8
   <211> 23
   <212> DNA
   <213> Artificial Sequence
<220>
   <223> CD37 reverse primer
<400> 8
   aactgtgcat agtcccaact ctc 23
<210> 9
   <211> 23
   <212> DNA
   <213> Artificial Sequence
<220>
   <223> CD81 forward primer
<400> 9
   ttctacgtgg gcatctacat tct 23
<210> 10
   <211> 23
   <212> DNA
   <213> Artificial Sequence
<220>
   <223> CD81 reverse primer
<400> 10
   gctgttcctc agtatggtgg tag 23
<210> 11
   <211> 23
   <212> DNA
   <213> Artificial Sequence
<220>
   <223> CD151 forward primer
<400> 11
   tgcctcaagt acctgctctt tac 23
<210> 12
   <211> 23
   <212> DNA
   <213> Artificial Sequence
<220>
   <223> CD151 reverse primer
<400> 12
   ctgactggtg gtatctcttg acc 23
<210> 13
   <211> 24
   <212> DNA
   <213> Artificial Sequence
<220>
   <223> KAI1(CD82) forward primer type I
<400> 13
   cactacaact ggacagagaa cgag 24
<210> 14
   <211> 25
   <212> DNA
   <213> Artificial Sequence
<220>
   <223> KAI1(CD82) reverse primer type I
<400> 14
   tgtagtcttc agaatgaatg taccg 25
<210> 15
   <211> 20
   <212> DNA
   <213> Artificial Sequence
<220>
   <223> Primer A
<400> 15
   gggtccccta ggaaattcaa 20
<210> 16
   <211> 25
   <212> DNA
   <213> Artificial Sequence
<220>
   <223> Primer B
<400> 16
   atgatgcaga tgttctctca gggtg 25
<210> 17
   <211> 22
   <212> DNA
   <213> Artificial Sequence
<220>
   <223> Primer C
<400> 17
   acaggggact caccctacaa gg 22
<210> 18
   <211> 23
   <212> DNA
   <213> Artificial Sequence
<220>
   <223> P21 forward primer
<400> 18
   gagaacggtg gaactttgac ttc 23
<210> 19
   <211> 24
   <212> DNA
   <213> Artificial Sequence
<220>
   <223> P21 reverse primer
<400> 19
   gtgatagaaa tctgtcaggc tggt 24
<210> 20
   <211> 22
   <212> DNA
   <213> Artificial Sequence
<220>
   <223> P27 forward primer
<400> 20
   catgaagaac taacccggga ct 22
<210> 21
   <211> 22
   <212> DNA
   <213> Artificial Sequence
<220>
   <223> P27 reverse primer
<400> 21
   cagagtttgc ctgagaccca at 22
<210> 22
   <211> 23
   <212> DNA
   <213> Artificial Sequence
<220>
   <223> P57 forward primer
<400> 22
   agatctgacc tcagacccaa ttc 23
<210> 23
   <211> 22
   <212> DNA
   <213> Artificial Sequence
<220>
   <223> P57 reverse primer
<400> 23
   gctcttgatt ctcgtcctgc tc 22
<210> 24
   <211> 18
   <212> DNA
   <213> Artificial Sequence
<220>
   <223> KAI1(CD82) forward primer type II
<400> 24
   gcctgggact acgtgcag 18
<210> 25
   <211> 21
   <212> DNA
   <213> Artificial Sequence
<220>
   <223> KAI1(CD82) reverse primer type II
<400> 25
   cctcgttctc tgtccagttg t 21
<210> 26
   <211> 25
   <212> DNA
   <213> Artificial Sequence
<220>
   <223> TGF-beta1 forward primer
<400> 26
   cggactacta tgctaaagag gtcac 25
<210> 27
   <211> 26
   <212> DNA
   <213> Artificial Sequence
<220>
   <223> TGF-beta1 reverse primer
<400> 27
   gagtttgtta tctttgctgt cacaag 26
<210> 28
   <211> 23
   <212> DNA
   <213> Artificial Sequence
<220>
   <223> TGF-beta R2 forward primer
<400> 28
   catcttctac tgctaccgtg tcc 23
<210> 29
   <211> 23
   <212> DNA
   <213> Artificial Sequence
<220>
   <223> TGF-beta R2 reverse primer
<400> 29
   atgttctcat gcttcaggtt gat 23

## Claims

1. Use of a composition for regulating the cell cycle of hematopoietic stem cells, the composition comprising:
a KAI1(CD82) polypeptide or a gene encoding the same, and
a DARC polypeptide or a gene encoding the same.,
wherein the use is in vitro.

2. The use of claim 1, wherein the KAI1 polypeptide consists of amino acids represented by SEQ ID NO: 1 or SEQ ID NO: 2.

3. The use of claim 2, wherein the DARC polypeptide consists of amino acids represented by SEQ ID NO: 3 or SEQ ID NO: 4.

4. The use of claim 1, wherein the hematopoietic stem cells are long-term hematopoietic stem cells (LT-HSCs), short-term hematopoietic stem cells (ST-HSCs), or multipotent progenitors (MPPs).

5. A method for collecting quiescent hematopoietic stem cells, comprising:
increasing KAI1 expression in hematopoietic stem cells,
wherein the hematopoietic stem cells are long-term hematopoietic stem cells (LT-HSCs), short-term hematopoietic stem cells (ST-HSCs), or multipotent progenitors (MPPs), wherein the hematopoietic stem cells are cultured with a rhDARC, DARC positive monocyte, DARC positive macrophage, or DARC positive macrophage culture solution to increase or maintain KAI1 expression.

## Patentansprüche

1. Verwendung einer Zusammensetzung zum Regulieren des Zellzyklus von hämatopoetischen Stammzellen, wobei die Zusammensetzung umfasst:
ein KAI1(CD82)-Polypeptid oder ein Gen, das es codiert, und
ein DARC-Polypeptid oder ein Gen, das es codiert,
wobei die Verwendung in vitro erfolgt.

2. Verwendung nach Anspruch 1, wobei das KAI1-Polypeptid aus Aminosäuren besteht, die durch SEQ ID NR.: 1 oder SEQ ID NR.: 2 dargestellt sind.

3. Verwendung nach Anspruch 2, wobei das DARC-Polypeptid aus Aminosäuren besteht, die durch SEQ ID NR.: 3 oder SEQ ID NR.: 4 dargestellt sind.

4. Verwendung nach Anspruch 1, wobei die hämatopoetischen Stammzellen hämatopoetische Langzeitstammzellen (LT-HSCs), hämatopoetische Kurzzeitstammzellen (ST-HSCs) oder multipotente Vorläufer (MPPs) sind.

5. Verfahren zum Sammeln von ruhenden hämatopoetischen Stammzellen, das umfasst:
Erhöhen der KAI1-Expression in hämatopoetischen Stammzellen,
wobei die hämatopoetischen Stammzellen hämatopoetische Langzeitstammzellen (LT-HSCs), hämatopoetische Kurzzeitstammzellen (ST-HSCs) oder multipotente Vorläufer (MPPs) sind, wobei die hämatopoetischen Stammzellen mit einem rhDARC, einem DARC-positiven Monozyten, einem DARC-positiven Makrophagen oder einer Kulturlösung mit DARC-positiven Makrophagen kultiviert werden, um die KAI1-Expression zu erhöhen oder aufrechtzuerhalten.

## Revendications

1. Utilisation d'une composition pour réguler le cycle cellulaire de cellules souches hématopoïétiques, la composition comportant :
un polypeptide KAI1(CD82) ou un gène codant celui-ci, et
un polypeptide DARC ou un gène codant celui-ci,
dans laquelle l'utilisation s'effectue in vitro.

2. Utilisation selon la revendication 1, dans laquelle le polypeptide KAI1 est constitué d'acides aminés représentés par la SEQ ID N° 1 ou la SEQ ID N° 2.

3. Utilisation selon la revendication 2, dans laquelle le polypeptide DARC est constitué d'acides aminés représentés par la SEQ ID N° 3 ou la SEQ ID N° 4.

4. Utilisation selon la revendication 1, dans laquelle les cellules souches hématopoïétiques sont des cellules souches hématopoïétiques à long terme (LT-HSC), des cellules souches hématopoïétiques à court terme (ST-HSC) ou des progéniteurs multipotents (MPP).

5. Procédé pour collecter des cellules souches hématopoïétiques dormantes, comportant l'étape consistant à :
augmenter l'expression de KAI1 dans les cellules souches hématopoïétiques, dans lequel les cellules souches hématopoïétiques sont des cellules souches hématopoïétiques à long terme (LT-HSC), des cellules souches hématopoïétiques à court terme (ST-HSC) ou des progéniteurs multipotents (MPP), dans lequel les cellules souches hématopoïétiques sont mises en culture avec un rhDARC, un monocyte positif au DARC, un macrophage positif au DARC, ou une solution de culture de macrophages positifs au DARC pour augmenter ou maintenir l'expression de KAI1.
